(19) Europäisches Patentamt European Patent Office Office européen des brevets

(11) **EP 3 817 690 B1**

(12) **EUROPEAN PATENT SPECIFICATION**

(45) Date of publication and mention of the grant of the patent:
**28.08.2024 Bulletin 2024/35**

(21) Application number: **19737072.9**

(22) Date of filing: **04.07.2019**

(51) International Patent Classification (IPC):
***A61F 2/46*** (2006.01)

(52) Cooperative Patent Classification (CPC):
**A61F 2/4603;** A61F 2/4607; A61F 2/4609;
A61F 2/461; A61F 2/4612; A61F 2002/4671;
A61F 2002/4681; A61F 2002/4689

(86) International application number:
**PCT/EP2019/068036**

(87) International publication number:
**WO 2020/008003 (09.01.2020 Gazette 2020/02)**

(54) **ENHANCER ELEMENT**

VERSTÄRKERELEMENT

ÉLÉMENT D'AMÉLIORATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.07.2018 GB 201810972**

(43) Date of publication of application:
**12.05.2021 Bulletin 2021/19**

(73) Proprietor: **Katholieke Universiteit Leuven
KU Leuven Research & Development
3000 Leuven (BE)**

(72) Inventors:
• **DENIS, Kathleen
3220 Kortrijk-Dutsel (BE)**
• **DESMET, Wim
3051 Sint-Joris-Weert (BE)**

• **GOOSSENS, Quentin
3200 Aarschot (BE)**
• **LEURIDAN, Steven
3000 Leuven (BE)**
• **MULIER, Michiel
3370 Boutersem (BE)**
• **PASTRAV, Leonard Cezar
3000 Leuven (BE)**
• **VANDER SLOTEN, Jos
3190 Boortmeerbeek (BE)**

(74) Representative: **Winger
Mouterij 16 bus 101
3190 Boortmeerbeek (BE)**

(56) References cited:
**EP-A1- 3 260 088          WO-A1-2015/187876
WO-A1-2016/187305     US-A1- 2015 282 856**

EP 3 817 690 B1

**Description**

**Field of the Invention**

The present invention relates to an enhancer element, in particular an enhancer element for use in assessment of an implant-bone system.

**Background**

In surgical procedures to replace musculoskeletal joints, the initial stability of an orthopaedic implant is important for the long term success of the artificial joint. The quality of contact can be influenced by one or more of size of contact areas between the implant and the bone, distribution of contact areas, and press-fit between the bone and the implant. However, intraoperative assessment of the initial stability of an implant can be challenging, and surgeons tend to rely on their experience and base their evaluation on subjective tactile, visual, and audio feedback.

This can be a particular problem in press fitted implants, which are inserted by means of impaction blows by a hammer. In such procedures it can be difficult to precisely determine the optimal end point of insertion, which can influence the outcome of the joint replacement. Fewer impaction blows than necessary can result in an unstable implant, whereas more blows than necessary can result in fracture of the bone into which the implant is inserted. Cristofolini et al, Med. Eng. Phys. June 2006; 28(5):475-82 describes a method in which the angle of the stem/femur rotation under torsion and the torque are acquired and compared in real-time to a pre-set threshold. However, applying such torque can stress the femur and result in damage to the bone and its surroundings.

Furthermore, methods using impaction blows as the excitation event for a measurement may be less sensitive as properties of the implant-bone system may change during the measurement and no repeated measurement may be possible.

Among these methods, document WO2015/187876 A1 describes a procedure of positioning an implant in a bone, based on high-fidelity audio recordings of hammer hits during implant installation. The comparison of the frequency bands with a database can be used to instruct a user regarding fit of the instrument within a bone.

Document EP3260088 A1 describes a similar approach, based frequency analysis of cumulative data obtained from successive impacts during assembly between a prosthetic component and a bone.

Document US2017/0196710 A1 shows a device, such as a modified sledgehammer or cockup gun, for introducing prosthesis in bone cavities with improved force alignment, as well as sensors for detecting changes in pitch when the implant bottoms out. This type of device is mainly useful for introducing acetabular cups, because for other types of interventions the prosthesis does not necessarily reach the bottom of the bone.

**Summary**

According to a first aspect of the present invention, there is provided a mechanical enhancer element as defined in claim 1.

During a measurement of a vibrational mode, the vibrational response of the enhancer-implant-bone system provides information about the stiffness of the enhancer-implant-bone system.

It is an advantage of embodiments of the present invention that properties of an interface or contact region between the implant and bone, such as the stability or fixation of the implant in the bone, can be assessed with increased sensitivity. It is a further advantage of embodiments of the present invention that such properties can be measured using an excitation of greatly reduced physical force, that does not significantly alter the stability of the implant and therefore the measurements can be repeated as many times as needed in order to reduce the effect of the varying environmental noise. It is a further advantage of embodiments of the present invention that the potential for damage to the bone may be detected before such damage occurs. It is a further advantage of embodiments of the present invention that the enhancer can provide an improved signal-to-noise ratio. It is a further advantage of embodiments of the present invention that sensors are not required to be placed directly on the implant, avoiding the requirement to sterilize electronic components. It is a further advantage of embodiments of the present invention that implant fixation can be measured using an element which does not need to be removed between measurements .

The first set of vibrational modes may comprise a first vibrational mode, the second set of vibrational modes may comprise a second vibrational mode corresponding to the first vibrational mode, and the second vibrational mode may have a lower frequency than the first vibrational mode.

The implant may have an implant mass and the enhancer element may have an enhancer element mass similar to the implant mass, within 10% over or under the implant mass for instance.

The implant may have an implant mass and the enhancer element may have an enhancer element mass substantially equal to the implant mass.

The implant may have an implant first resonance frequency and the enhancer element may have an enhancer element first resonance frequency which is substantially equal to the implant first resonance frequency.

The enhancer element may comprise an excitation element configured to provide a vibrational excitation to the enhancer-implant-bone system.

The enhancer element may comprise an excitation element configured to provide a vibro-acoustic excitation to the enhancer-implant-bone system.

The frequency range may include frequencies from 0 Hz to 2,5 kHz.

The frequency range may include frequencies from 0 Hz to 5 kHz.

The enhancer element may comprise at least one sensor element disposed on the enhancer element configured to detect a vibrational response of the enhancer-implant-bone system.

The implant may have an implant mechanical impedance, and the enhancer may have an enhancer mechanical impedance which is substantially the same as the implant mechanical impedance.

The implant may have an outer surface and the bone may have a cavity for receiving the implant, the cavity having an inner surface; a contact region may be defined by a region of contact between the implant outer surface and the cavity inner surface; and the second set of vibrational modes may include at least one vibrational mode having an anti-node within the contact region.

The implant may be a cementless implant.

The implant may be a cemented implant.

According to a second aspect of the present invention there is provided a system for intraoperative assessment of insertion of an orthopaedic implant comprising an enhancer element according to the first aspect and at least one detector configured to receive a vibrational signal from the enhancer element.

According to a third aspect of the present invention there is provided a system for intraoperative assessment of insertion of an orthopaedic implant comprising an enhancer element according to the first aspect and at least one detector configured to receive an acoustic signal from the enhancer element.

The at least one detector may comprise at least one microphone.

## Brief Description of the Drawings

Certain embodiments of the present invention will now be described, by way of example, with reference to the accompanying drawings, in which:

Figure 1 is a schematic perspective view of a first enhancer element according to embodiments of the present invention;

Figure 2 is a schematic perspective view of an enhancer element according to embodiments of the present invention coupled to an orthopaedic implant;

Figure 3a is a schematic perspective view of an implant disposed in a bone;

Figure 3b is a schematic cross section of a portion of a bone and an implant disposed in a cavity in the bone;

Figure 4 is a schematic perspective view of an enhancer element according to embodiments of the present invention coupled to an orthopaedic implant, the implant being disposed in a bone;

Figure 5a is a plot of frequency response function amplitudes for the insertion process of a cementless implant into a replicate composite femur;

Figure 5b shows the progression of subsidence of an implant for the insertion process of a cementless implant into a replicate composite femur as measured using a caliper;

Figure 5c shows the cross-correlation function shift as a function of insertion step transition for the insertion process of a cementless implant into a replicate composite n;

Figure 5d shows the modification index for a frequency band including high frequency information (100-4500Hz) as a function of insertion step transition for the insertion process of a cementless implant into a replicate composite femur;

Figure 5e shows the modification index for a frequency band in a low frequency range (100-750 Hz) as a function of insertion step transition for the insertion process of a cementless implant into a replicate composite femur;

Figure 6a is a plot of frequency response function amplitudes for the insertion process of a cementless implant into a cadaveric femur, as measured using an enhancer element according to embodiments of the present invention;

Figure 6b shows the progression of subsidence of an implant for the insertion process of a cementless implant into a cadaveric femur as measured using a caliper;

Figure 6c shows the cross-correlation function shift as a function of insertion step transition for the insertion process of a cementless implant into a cadaveric femur

Figure 6d shows the modification index for a frequency band including high frequency information (100-4500Hz) as a function of insertion step transition for the insertion process of a cementless implant into a cadaveric femur;

Figure 6e shows the modification index for a frequency band in a low frequency range (100-750 Hz) as a function of insertion step transition for the insertion process of a cementless implant into a cadaveric femur;

Figure 7a illustrates the division of a bone-implant contact zone into 24 contact zones of equal length;

Figure 7b shows the evolution of resonance frequencies of a cementless bone-implant system during insertion of the implant;

Figure 8a illustrates the third ML mode shapes of the bone implant system, with a resonance frequency of approximately 2kHz at contact zone six;

Figure 8b is a plot of the resonance frequency of the second and third ML modes as a function of increasing contact zone;

Figure 8c illustrates the second ML mode shapes of the bone implant system with a resonance frequency of approximately 1kHz at contact zone six;

Figure 9a illustrates the second ML bending mode of a bone-implant system;

Figure 9b illustrates the third ML bending mode of a bone-implant system;

Figure 9c is a plot of the resonance frequency as a function of contact zone for the second and third ML bending zones;

Figure 10a is a schematic cross-section of a bone-implant system indicating the relative positioning of the calcar zone (at 6.7%) and the full proximal zone;

Figure 10b illustrates the deformed bending (B) and longitudinal (L) mode shapes and corresponding MSED distribution of a simplified beam model. The location on the beam at which the element stiffness was changed, was progressively moved from a position located at 0.1% of total length to a position located at 50% of total length;

Figure 10c is a table presenting the percentage change in resonance frequency for the first 13 resonance frequencies, as a function of position on the beam at which element stiffness was changed;

Figure 11a is a schematic perspective view of an enhancer element of the 'beam' form according to embodiments of the present invention, the enhancer element being coupled to an implant;

Figure 11b illustrates the first bending mode of the implant-enhancer system of Figure 11a;

Figure 11c illustrates the second bending mode of the implant-enhancer system of Figure 11a;

Figure 11d is a schematic perspective view of an enhancer element of the 'delta' form according to embodiments of the present invention, the enhancer element being coupled to an implant;

Figure 11e illustrates the first bending mode of the implant-enhancer system of Figure 11d;

Figure 11f illustrates the second bending mode of the implant-enhancer system of Figure 11d;

Figure 12a is a schematic perspective view of a finite element model of an implant inserted into a bone;

Figure 12b is a schematic perspective view of a finite element model of an enhancer element of the beam type coupled to an implant inserted into a bone;

Figure 12c is a schematic perspective view of a finite element model of an enhancer element of the delta type coupled to an implant inserted into a bone;

Figure 13a illustrates an implant along with the frequency response function amplitudes of the implant within a bone as a function of frequency for proximal loosened and fully fixed states and its Pearson coefficient as a function of frequency for a range of modal damping values;

Figure 13b illustrates an enhancer element of the beam type coupled to an implant along with the frequency response function amplitudes of a system comprising the enhancer and implant wherein the implant is disposed in a bone, as a function of frequency for proximal loosened and fully fixed states and its Pearson coefficient as a function of frequency for a range of modal damping values;

Figure 13c illustrates an enhancer element of the delta type coupled to an implant along with the frequency response function amplitudes of a system comprising the enhancer and implant wherein the implant is disposed in a bone, as a function of frequency for proximal loosened and fully fixed states and its Pearson coefficient as a function of frequency for a range of modal damping values;

Figure 14 illustrates the MSED distribution for implant mode shapes corresponding to a range of resonance frequencies in the antero-posterior and medio-lateral planes;

Figure 15a is a visual matrix representation of MAC values for bone-implant-enhancer systems comprising an enhancer according to embodiments of the present invention;

Figures 15b and 15c are plots of the frequency response functions of enhancer elements of the beam type and the delta type, respectively;

Figures 15d and 15e illustrate bending modes of bone-implant-enhancer systems for enhancers of the beam time and the delta type, respectively;

Figure 16a illustrates a frequency response function and bending modes in the ML direction for the beam model;

Figure 16b illustrates a frequency response function and bending modes in the ML direction for the delta model;

Figure 17a is a photograph of an enhancer element of the beam form according to embodiments of the present invention;

Figure 17b is a photograph of an enhancer element of the delta form according to embodiments of the present invention;

Figure 18 illustrates three experimental model configurations tested, including a reference bone-implant system, a bone-implant-enhancer system where the enhancer is of the beam form, and a bone-implant-enhancer system where the enhancer is of the delta form;

Figure 19a is a plot of implant subsidence as a function of insertion step for the bone-implant enhancer system where the enhancer is of the delta form;

Figure 19b is a plot of the frequency response function of the bone-implant enhancer system measured at various insertion steps where the enhancer is of the delta form;

Figure 20 shows the frequency response function for insertion steps 6, 7, and 8 in the low and high frequency ranges, and the PC and FRAC for the low and high frequency ranges, for the reference bone-implant system of Figure 18;

Figure 21 shows the frequency response function for insertion steps 6, 7, and 8 in the low and high frequency ranges, and the PC and FRAC for the low and high frequency ranges, for the bone-implant-enhancer system of Figure 18 wherein the enhancer is of the beam form;

Figure 22 shows the frequency response function for insertion steps 6, 7, and 8 in the low and high frequency ranges, and the PC and FRAC for the low and high frequency ranges, for the bone-implant-enhancer system of Figure 18 wherein the enhancer is of the delta form;

Figure 23 shows the PC and FRAC metrics as a function of insertion step transition, for the bone-implant-enhancer system of Figure 17 wherein the enhancer is of the delta form, in the frequency range 100-2500Hz;

Figure 24 is a schematic cross-section of a second enhancer element according to embodiments of the present invention, the second enhancer element being shown coupled to an implant, the implant being disposed in a bone;

Figures 25a-g show various perspective and cross-sectional views of an enhancer element according to embodiments of the present invention;

Figure 26 is a plot of FRF amplitudes of a bone-implant system (dashed line) and a bone-implant enhancer (solid line) system measured in the medio-lateral direction, for the enhancer of Figure 25;

Figure 27 is a plot of FRF amplitudes of a bone-implant system (dashed line) and a bone-implant enhancer (solid line) system measured in the antero-posterior direction, for the enhancer of Figure 25;

Figure 28a illustrates the undeformed and $3^{rd}$ bending mode of a bone-implant-enhancer system wherein the enhancer is a second enhancer element according to embodiments of the present invention;

Figure 28b illustrates the undeformed and $3^{rd}$ bending mode of a bone-implant system;

Figure 29 is a plot of the acoustic amplitude FRF for an implant insertion experiment, as measured in the AP direction to illustrate the change in vibro-acoustic behavior. The implant was inserted in 10 steps. The FRF's measured at step 3, 5, 8, and 10 are plotted as an illustration;

Figure 30 is a plot of the Frequency Assurance Criterion (FRAC) calculated between every insertion step for the insertion experiment of Figure 29;

Figure 31 is a plot of an acoustic output only spectrum of an implant insertion experiment measured in the AP direction illustrating the change in vibro-acoustic behavior. The implant was inserted in 16 steps. The FRFs measured at step 1, 4, 7, 10, 13 and 16 are plotted as an illustration;

Figure 32 is a plot of Pearson Correlation Coefficient (PCC) calculated between every two subsequent insertion steps for the experiment of Figure 31 using an output-only acoustic approach performed in the AP direction;

Figure 33 is a schematic diagram of a system according to embodiments of the present invention comprising an enhancer element which is couplable to an implant;

Figure 34 is a flow chart of a method according to embodiments of the present invention;

Figure 35 is a perspective view of a connecting configuration for coupling an enhancer element according to embodiments of the present invention to an implant;

Figure 36 is a side view of a connecting configuration for coupling an enhancer element according to embodiments of the present invention to an implant, and a screwdriver;

Figure 37 is a schematic plan view of an enhancer element according to embodiments of the present invention comprising a radiating surface.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

## Detailed Description of Certain Embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

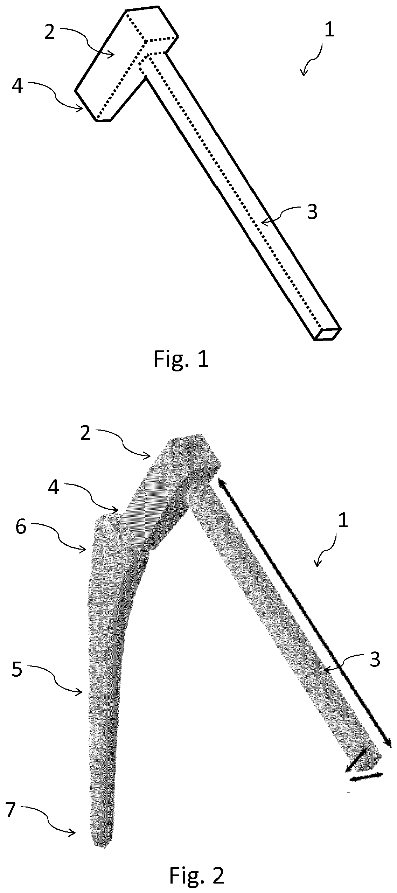

Referring to Figure 1, a first enhancer element 1 according to embodiments of the present invention is shown. The first enhancer element 1 is a mechanical enhancer element. The first enhancer element 1 comprises a first implant coupling portion 2 and a first matching portion 3. The first implant coupling portion 2 includes a first end portion 4 which is mechanically couplable to an orthopaedic implant (Figure 2). Referring to Figure 2, the first enhancer element 1 is configured to couple mechanically to an orthopaedic implant 5 at the first end portion 4 of the first enhancer element 1. The mechanical coupling may be assisted by, for example, a screw or bolt attachment (not shown).

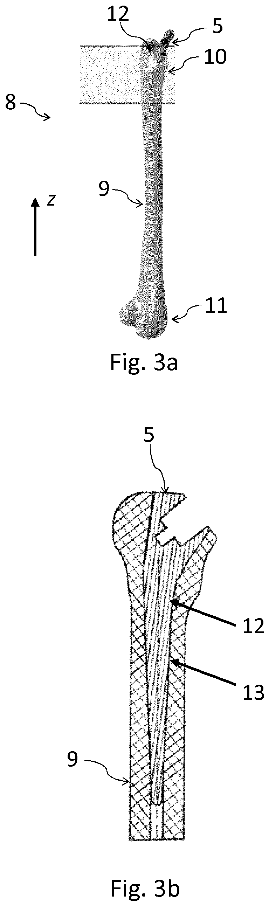

The orthopaedic implant 5 comprises a first end 6 and a second, opposite end 7. During a joint replacement procedure, the orthopaedic implant 5 is introduced to a bone (not shown) by positioning the second end 7 of the implant 5 in a prepared cavity in the bone (Figure 3) and applying one or more impaction blows at the first end 6, so as to provide an impaction force. The first enhancer element 1 is configured to couple to the implant 5 at the first end 6 of the implant 5. This allows the first enhancer element 1 to be easily removable from the implant 5 without disturbance to the implant 5 or the bone (Figure 3).

Moreover, the placement of the enhancer element 1 may not disturb insertion. It may leave enough space on the first end 6 of the implant 5 so that implant 5 can be inserted, for example by impaction blows provided directly on the first end 6 of the implant 5. This is shown in Figures 3b, 18, 24 and 33, for example. In some embodiments, the enhancer element 1 is placed on the neck of the implant 5.

Referring to Figures 3a and 3b, a bone-implant system 8 is shown comprising an implant 5 inserted into a bone 9. The bone 9 extends between a first end 10 and a second end 11 in a first direction z. The implant 5 is inserted in the first direction z into a prepared cavity 12 in the bone 9 at the first end 10. The bone-implant system comprises a contact region 13 which is a region of contact between an outer surface of the implant 5 and an inner surface of the bone 9 in the cavity 12.

During installation of the implant 5, impaction blows are provided at the first end 6 of the implant 5. The stability of the implant 5 within the bone 9 can be influenced by the quality of contact in the contact region 13. Most of the contact is established in the final steps of the insertion process. Implant movement however is very limited in these last few steps and is generally in the order of magnitude of millimeters or less. The enhancer 1 according to embodiments of the present invention allows to provide a structural health monitoring system capable of assessing the stability of a bone-implant system which is sensitive to changes in stiffness and/or damping in the contact region. Such changes are reflected in the vibrational behavior of a system formed by the enhancer 1, the implant 5, and the bone 9.

In some embodiments the enhancer is sensitive to stiffness and/or damping in a sub-region of the contact region, for example a sub-region which is closer to the first end 10 or the second end 11 of the bone. Contact build-up in such a sub-region may be important for stability of the bone-implant system. The location, size, and shape of the sub-region may depend on the type of implant (for example a primary implant, a revision implant). In some embodiments, the sub-region is a region which extends for approximately one-third of the bone length and is at the first end 10 of the bone. In some embodiments, the sub-region may be chosen as a region which is more sensitive to the buildup of contact than other sub-regions of the contact region, for example a region in which at least one vibrational mode has an anti-node.

The sub-region may be chosen to be appropriate for the particular type of implant in use. An implant can be designed to build up its stability at a specific region of contact between the implant and the bone. For example, a primary implant may be designed to build up its stability in a proximal region due to the tapered geometry of the bone at the proximal zone, which allows a good press-fit of the implant, and the sub-region may preferably be chosen to include the proximal zone. During primary total hip arthroplasty there is generally enough healthy bone available in the proximal zone. For a revision implant, there may be significant bone loss at the proximal region and so press fit may need to be provided at a more distal zone, and the sub-region may be chosen to be further from the first end 10 of the bone than for a primary implant.

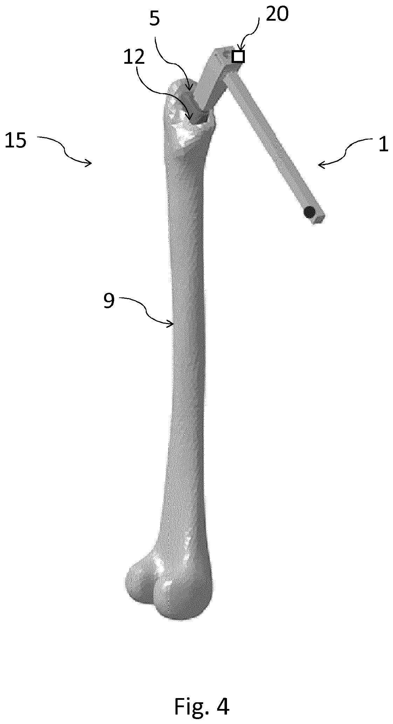

Referring to Figure 4, an enhancer-implant-bone system 15 is shown comprising the implant 5 inserted into the bone 9 and the first enhancer element 1 coupled to the implant 5. The enhancer-implant-bone system 15 comprises a contact region 13 as described in relation to Figures 3a and 3b.

In use, the enhancer 1 is coupled to the implant 5 and is excited by applying a force to the enhancer 1. Such a force may be applied by, for example, applying an oscillating signal generated by a shaker or by applying one or several light hammer impacts to the matching portion 3 or the implant coupling portion 4 of the enhancer 1. The direction and location of the application of force may be chosen in dependence upon the vibrational modes to be measured, for example the force may be applied at or near an anti-node of a mode to be measured. The force may be applied in the same plane as the plane of vibration of a mode to be measured. The mode to be measured is preferably a mode having an anti-node at or near a contact region in which contact builds up for good fixation of the implant.

The resulting vibrational response of the enhancer-implant-bone system 15 can be detected using, for example, one or more sensors 20 which may be vibrational sensors. The one or more sensors may comprise an accelerometer, a velocity sensor disposed on the enhancer 1, a microphone, a laser vibrometer. A vibrational sensor is preferably positioned at or near an anti-node of a vibrational node to be measured.

The vibrational response of the enhancer-implant-bone system 15 provides information about the stiffness of the system 15. The vibrational response can be affected by one or more factors such as tissue surrounding the bone. The stiffness can be affected by the degree to which the implant 5 is inserted into the bone 9, the condition of the bone 9, the presence of fractures in the bone 9, the degree of proximal contact between the bone 9 and the implant 5 in the proximal region. Thus by monitoring the vibrational response of the system 15 during installation of the implant 5, by exciting the enhancer 1 (e.g. by applying a force on the enhancer as explained earlier) after one or more impaction blows for installing the implant 5, the surgeon can decide whether an optimal fitting of the implant has been achieved and whether further impaction blows to the implant 5 would be likely to cause damage or fractures to the bone 9. It is noted that the enhancer is configured to be excited with a force that is usually not in the same direction and/or with the same magnitude as the force applied during impaction blows for installing the implant. The nature (e.g. amplitude and/or direction) of the force of an impaction blow is different from the excitation of the enhancer 1 during a vibrational measurement. The enhancer is adapted to receive an excitation by a force which may have different direction from the force applied with an impaction blow; additionally the magnitude of the force of an impaction blow is usually much higher than the excitation force of the enhancer. Moreover, the impaction blow is provided on the implant, rather than on the enhancer.

*Examples*

Example embodiments of a first enhancer 1 are herein described. However, it will be understood that the present invention is not limited thereto, and other embodiments are possible within the scope of the present invention.

A bone-implant system was investigated through experiment and modelling to determine its vibrational response, also referred to as frequency response function.

A composite femur model (Sawbones model 3403 (size medium), Sawbones Europe AB, Malmö, Sweden) and a frozen embalmed human cadaveric femur model were prepared by an experienced surgeon for implantation of an uncemented Mathys Twinsys size 12 implant (Mathys Medical, Bettlach, Switzerland) using manufacturer provided standard instruments. The cadaveric bone model was thawed overnight prior to the experiment. After preparation, the implant was hammered in by the surgeon. After every hammer blow, the depth of the implant was measured using a digital caliper and a frequency response function (FRF) was collected. The FRF was measured on the neck of the implant in the antero-posterior (AP) direction. The excitation was performed by impaction using a modal hammer (PCB 086C03) and the acceleration response was measured using a lightweight accelerometer (PCB A352A24). The excitation and measurement locations were opposite of each other on the neck of the implant, hence a direct FRF was measured. Data acquisition and conditioning were performed using a spectral analyzer (LMS SCADAS Mobile, Siemens PLM Software, Leuven, Belgium) and corresponding software (LMS Test Lab). The sampling frequency was set to 20.48 kHz, the frequency resolution was 0.625 Hz. Four resonance frequencies with the highest amplitude in the FRF and their damping factors were extracted using the Polymax algorithm in a range of 100-4500 Hz. All other data processing was performed in Matlab (Matlab, Natick, MA, USA). Free-free conditions were simulated as these boundary conditions were proven to closely mimic the in vivo situation.

Two metrics were used to assess the change in FRFs between insertion steps; the Pearson's correlation (PC) and the cross-correlation function (CCF). The shift associated with the highest CCF value was reported.

$$PC = \frac{\sum_{f=a}^{b}(|H(f)|_{N-1}-\overline{|H(f)|}_{N-1})(|H(f)|_N-\overline{|H(f)|}_N)}{\sqrt{\sum_{f=a}^{b}(|H(f)|_{N-1}-\overline{|H(f)|}_{N-1})^2}\sqrt{\sum_{f=a}^{b}(|H(f)|_N-\overline{|H(f)|}_N)^2}}$$

Where $|H(f)|N$ is the amplitude of the FRF obtained at insertion step N. The PC is calculated in a frequency range from $a$ to $b$.

$$CCF(k) = \frac{\sum_{f=a}^{b}(|H(f)|_{N-1}-\overline{|H(f)|}_{N-1})(|H(f+k)|_N-\overline{|H(f)|}_N)}{\sqrt{\sum_{f=a}^{b}(|H(f)|_{N-1}-\overline{|H(f)|}_{N-1})^2}\sqrt{\sum_{f=a}^{b}(|H(f+k)|_N-\overline{|H(f)|}_N)^2}}$$

for $k = 0, +- 1\Delta f, +- 2\Delta f...$

Higher frequencies generally tend to be more sensitive to changes of the bone-implant system during the insertion process, for example as described in Qi et al, "How much can a vibrational diagnostic tool reveal in total hip arthroplasty loosening?", Clinical Biomechanics 18 (5) 444-458. To exemplify this, two frequency bands were selected to calculate the PC metric; one in a low frequency range (LF: 100-750 Hz) and one including the higher frequency portion of the FRF (HF: 100-4500 Hz).

A Modification Index (MI), which varies between zero and one, is calculated as:

Modification Index = $1 - PC$

The term 'Modification Index' is preferred over the more commonly used 'Damage Index' (DI), as the insertion process is more a procedure of modifying and building the interface between bone and implant rather than damaging it. Low values of the Modification Index indicate little change is effected to the bone-implant system between insertion steps.

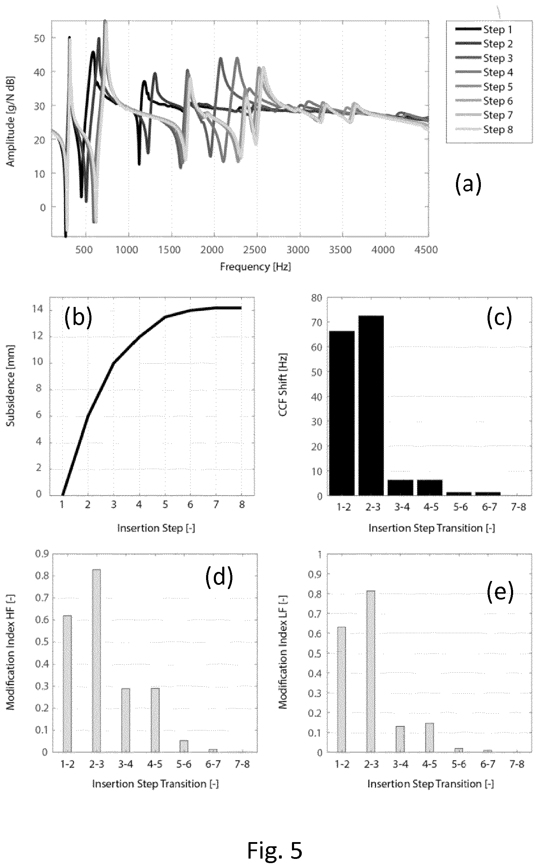

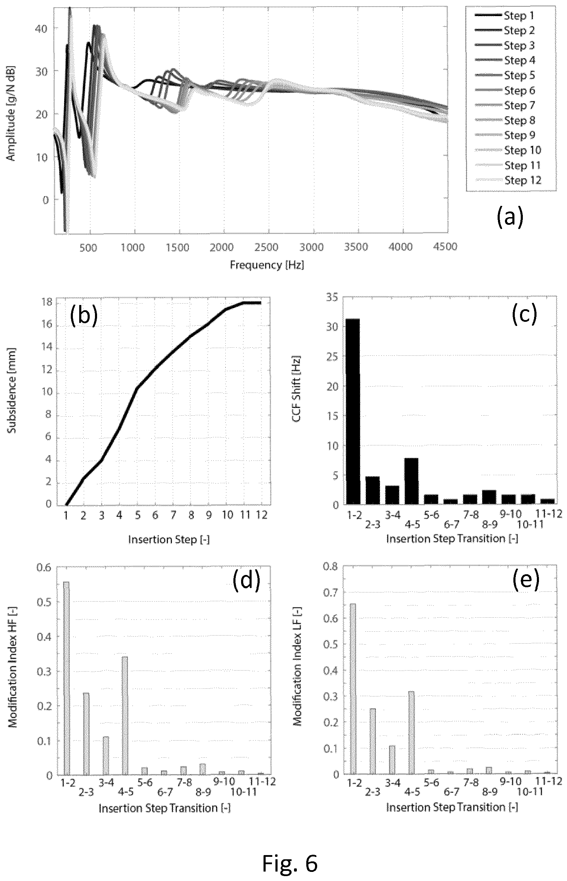

Figures 5 and 6 summarize the results from these insertion experiments. The implant needed eight steps to reach a fully inserted position for the composite bone model (Figure 5) and subsided approximately 14 mm during the insertion process, compared to 12 steps and a total subsidence of 18 mm for the cadaveric bone model (Figure 6). Comparable in both insertions was that the majority of the subsidence is realized in the first steps and with rather limited subsidence in the last few steps.

Apparent convergence of the FRF feature as the endpoint of insertion nears can be observed in Figures 5a and 6a.

*Table 1: Resonance frequencies and modal damping coefficients extracted from the experimental FRFs for the replicate composite femur model and the cadaveric femur model*

**Sawbones Femur**

| | Step 1 | Step 2 | Step 3 | Step 4 | Step 5 | Step 6 | Step 7 | Step 8 |
|---|---|---|---|---|---|---|---|---|
| Frequency [Hz] | | | | | | | | |
| | 306.6 | 309.5 | 312.1 | 312.4 | 313.4 | 313.5 | 313.9 | 313.8 |
| | 594.8 | 653.5 | 720.4 | 727.5 | 735.1 | 737.4 | 738.9 | 738.8 |
| | 1174.5 | 1298.5 | 1683.2 | 1695.7 | 1708.4 | 1714.7 | 1717.3 | 1717.2 |
| | 1652.2 | 1689.6 | 2071.7 | 2261.0 | 2516.9 | 2557.4 | 2571.2 | 2571.5 |
| Damping [%] | | | | | | | | |
| | 0.6% | 0.5% | 0.5% | 0.5% | 0.6% | 0.5% | 0.5% | 0.5% |
| | 0.9% | 0.7% | 0.7% | 0.7% | 0.6% | 0.6% | 0.6% | 0.6% |
| | 1.6% | 1.7% | 0.9% | 0.8% | 0.8% | 0.8% | 0.8% | 0.8% |
| | 1.1% | 0.8% | 1.0% | 1.2% | 0.8% | 0.9% | 0.9% | 0.8% |

**Cadaveric Femur**

| | Step 1 | Step 2 | Step 3 | Step 4 | Step 5 | Step 6 | Step 7 | Step 8 | Step 9 | Step 10 | Step 11 | Step 12 |
|---|---|---|---|---|---|---|---|---|---|---|---|---|
| Frequency [Hz] | | | | | | | | | | | | |
| | 252.0 | 262.9 | 281.1 | 283.2 | 289.6 | 290.8 | 291.7 | 293.3 | 295.5 | 296.8 | 298.3 | 298.7 |
| | 481.7 | 532.9 | 584.2 | 603.1 | 639.0 | 644.9 | 649.5 | 655.8 | 661.6 | 664.0 | 667.6 | 667.5 |
| | 1084.6 | 1184.4 | 1329.5 | 1398.7 | 1536.8 | 1559.9 | 1574.1 | 1591.9 | 1612.4 | 1622.4 | 1632.2 | 1631.4 |
| | 1623.6 | 1687.4 | 1818.8 | 1887.2 | 2132.2 | 2210.5 | 2265.4 | 2418.7 | 2460.0 | 2488.4 | 2497.2 | 2495.8 |
| Damping [%] | | | | | | | | | | | | |
| | 3.3% | 6.3% | 1.3% | 1.2% | 1.1% | 1.1% | 1.1% | 1.1% | 1.1% | 1.1% | 1.1% | 1.1% |
| | 6.1% | 6.0% | 3.1% | 3.0% | 3.2% | 3.1% | 3.1% | 3.0% | 3.1% | 3.1% | 3.2% | 3.2% |
| | 10.7% | 10.2% | 5.0% | 4.5% | 4.3% | 4.3% | 4.3% | 4.3% | 4.4% | 4.4% | 4.6% | 4.6% |
| | 8.0% | 7.1% | 4.9% | 4.8% | 5.3% | 6.0% | 5.1% | 5.7% | 5.1% | 6.3% | 6.1% | 6.3% |

Contrasting the results obtained for the MI when calculated for the band without and the band including the high frequency information, little differences are observed for the cadaveric bone model. The composite bone model shows similar values for the MI in the first three steps of the insertion, where the geometry of the bone-implant system is changing significantly, and then display a higher sensitivity in the HF range for the final five steps, where proximal contact between bone and implant is established. The main reason for this difference in results between the cadaveric and composite bone model is the difference in damping in the two systems. This is visible in the plotted FRFs of Figures 5a and 6a.

Although the progression of the vibrational behavior is similar for the composite and cadaveric bone models, the influence of damping on the measured FRFs is not, especially at higher frequencies (above 1000 Hz). The limited sensitivity of the lower frequency modes and the difference in damping between the composite and cadaveric bone model are also evidenced when comparing the respective resonance frequencies and modal damping parameters corresponding to the four highest amplitude peaks in the FRF (table 1). It is clear that higher frequency range shows higher sensitivity to bone-implant system changes, especially towards the end of the insertion process.

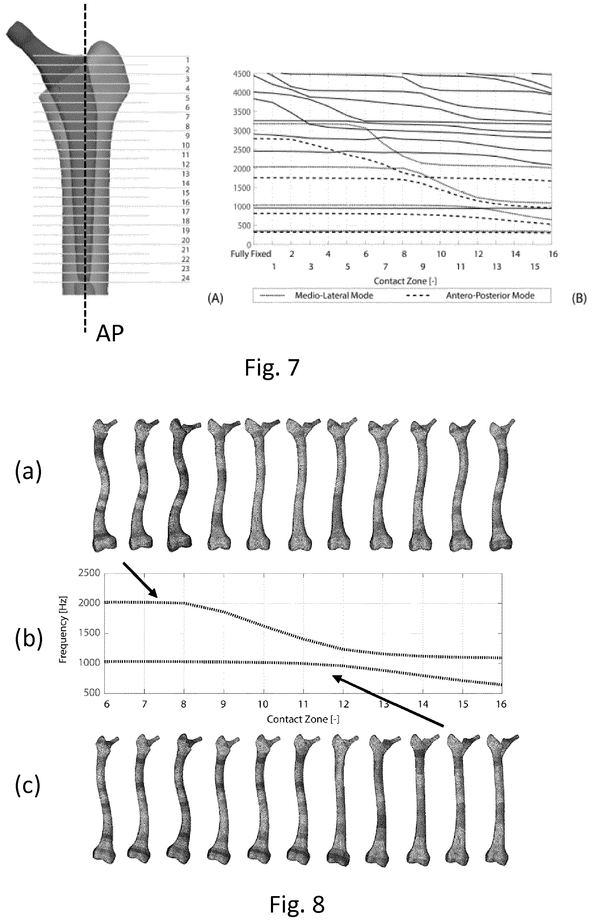

Simulations were also performed to investigate the effect of bone to implant contact changes on the vibrational behavior of a cementless bone-implant system. A model was used as described in Leuridan et al., Determination of replicate composite bone material properties using modal analysis Journal of the Mechanical Behavior of Biomedical Materials (66), art.nr. 51751-6161(16)30372-1, 12-18. Referring to Figure 7a, the region around the stem was divided in 24 contact zones of equal length. The numerical experiment simplifies the mechanics of the contact building process as it changes contact in discrete incremental steps around the implant, moving from bottom (zone 24) to top (zone one). Contact was established by equivalencing matching nodes on bone and implant surface, corresponding to a glued contact condition. In order to isolate the influence of the contact, the position of the implant relative to the bone was kept constant. At every step a modal analysis was performed calculating the mode shapes and resonance frequencies using MSC Nastran (MSC Nastran, MSC Software, Newport Beach, CA, USA). Pre -and post-processing of the models was done in LMS Virtual Lab (Siemens PLM Software, Leuven, Belgium).

Figure 7b shows the evolution of the resonance frequencies as the contact is changed around the implant. The x-axis lists the zones that are incrementally brought into contact. For example zone 16 on the x-axes presents the resonance frequencies for the case where contact is established between bone and implant in zones 24-16. Similarly for 15, for which contact is then established in zones 24-15 etc. Thus for every step one additional zone is added to the total region already in contact. For ease of interpretation, the following convention is assumed; Medio-Lateral (ML) mode shapes have most of their deformation in the frontal plane and Antero-Posterior (AP) mode shapes have most of their deformation in the sagittal plane. The frontal plane coincides with the plane of the cross section of Figure 7a. The sagittal plane is perpendicular to the frontal plane and includes the dashed line AP shown in Figure 7a.

The changes in resonance frequencies are reported in the range 10-4500 Hz, analogous to the experimental range. A few observations stand out. Firstly, when contact is changed in the proximal zone (contact zones 1-8), resonance frequencies below 2000 Hz are very little changed. Secondly, not all resonance frequencies change at the same time when contact is altered. For example, the resonance frequency associated with the third AP mode (with a resonance frequency around 1000 Hz at contact zone 16) shows an important change when contact zones 15-10 are brought into contact.

The resonance frequency associated with the fourth AP bending mode (resonance frequency around 1500 Hz at contact zone 16) is little influenced by this change. Resonance frequencies were tracked using the Modal Assurance Criterion (MAC [3]) in the 10-3000 Hz range. The evolution of the first four

AP and ML bending modes could thus be followed. Mode shapes above 3000 Hz generally displayed combined transverse, longitudinal and torsional behavior impeding adequate tracking.

The evolution of the resonance frequencies displays mode veering and crossing. Mode veering refers to the rapid approach of two eigenvalue branches when a variable system parameter is changing and that then veer away and diverge instead of cross. This phenomenon is strongest in weakly coupled systems, but can also manifest itself to a lesser extent in strongly coupled systems. The bone-implant system under study could be considered an example of the latter. Figure 8 illustrates in more detail what is happening to the mode shapes of the bone-implant system as the contact parameter changes and as a result the resonance frequencies approach each other (Figure 8b). The phenomenon is easiest to illustrate when moving from a well fixed contact situation (zones 6-24 in contact) to a more loosened situation (zones 16-24 in contact). The third ML bending mode (Figure 8a) starts at 2000 Hz and gradually evolves into the second bending ML mode (Figure 8c) as the contact area changes. This mode shape then starts to interact with the mode shape of the ML mode below (second bending mode at 1000 Hz), which in its turn veers down and will evolve into the first bending mode.

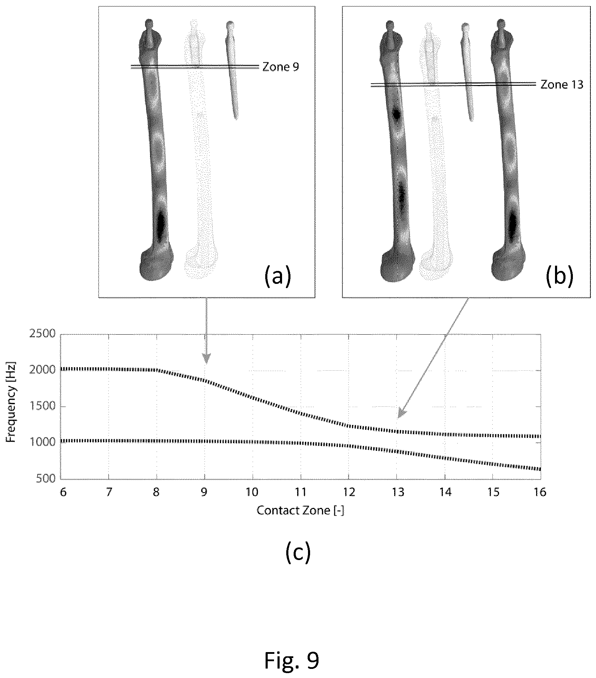

Figure 9 provides insight into when mode shapes start to be affected by contact changes between bone and implant. The Modal Strain Energy Density (MSED) is depicted for a fully fixed bone-implant system for the second (Figure 9a) and third (Figure 9b) ML bending mode. The MSED for the ith mode shape can be calculated as follows:

$$MSED_i = \frac{1}{2}\phi_i^T K \phi_i$$

where $\varphi_i$ is the ith mode shape vector and K is the global stiffness matrix, $(.)^T$ indicates the vector transpose. Reading the resonance frequency graph of Figure 9c from left to right, it can be seen that when a change is made to a zone that is strained by that particular mode shape (e.g. zone nine for the third mode shape), the resonance frequency is affected. Loosening of that zone locally reduces the stiffness of the bone-implant system. Mode shapes and resonance frequencies may only be affected by changes in stiffness if the zone where the stiffness changes is loaded by that particular mode shape. Stiffness changes to locations with the highest modal strain energy density can have the most impact on modal parameters of that mode. Local changes to the mass of the system can best be observable as a change in a mode shape and corresponding resonance frequency if that change is made at a location with maximum kinetic energy.

For the system under study, mode shapes and resonance frequencies are affected when a contact zone change nears a region with elevated MSED for a particular mode (e.g. zone nine for the third ML bending mode), is highest when it is in the vicinity of the maximum MSED for that mode (zones 10-12) and then levels of once it passes the point of maximum deflection for that mode. Subsequently it then enters into the MSED region of the mode below (zone 13 entering the region of elevated MSED of the second ML bending mode). The mode shape transitions in this example are most pronounced in zones 10-12 and are accompanied by important changes in resonance frequencies for that mode. It is clear from these results that lower frequency modes put little strain on the proximal zones and thus any changes in stiffness in this region due to contact build-up between bone and implant go largely unaffected.

These insights into the change of the vibrational response as an implant is inserted can be used to determine properties of an enhancer element according to embodiments of the present invention. An enhancer element according to embodiments of the present invention can have one or more advantages such as being compact, not requiring an assistant to perform the excitation, not requiring a modification of the implant, using a vibrational excitation that is separate from the impaction blows which can be of much less force than the impaction blows and thus not changing substantially the seating of the implant in the bone during measurement and thus allowing repeating of the measurements.

The dimensions and shapes described herein are understood to be examples only and the present invention is not limited thereto.

Referring again to Figure 1, the dimensions of the implant coupling portion 2 can be determined, for example, so as to avoid contact of the enhancer 1 with the patient's tissue which may lead to unintended energy dissipation, sensors and actuators which may be disposed in or on the enhancer 1 are preferably located at a distance that clears the thickness of the skin and subcutaneous tissue layers. The combined thicknesses of subcutaneous tissue and skin layers have been reported to range from 4.57 mm (SD 1.55) to 13.26 mm (SD 4.45) for male subjects, with the former for subjects with a BMI < 17 and the latter for subjects with a BMI above 25. Female subjects can have thicknesses which vary from 6.39 mm (SD 1.86) to 14.82 mm (SD 7.11) in soft tissue layer thickness with a similar BMI range. In order to comfortably surmount these soft tissues zones, the minimal length of the end portion 4 was set to 40 mm. Avoidance of the working zone of the surgeon and the requirement to stay clear of the soft tissue layers define the outer boundaries of the design space for the enhancer element.

The implant coupling portion 2 preferably should allow swift mounting and dismounting of the enhancer 1 without damaging the implant 5. The Profemur implant is fitted with an internal conical Morse taper and a hole with a M7 thread which can be used as a slot for the implant coupling portion of the enhancer. A torque wrench can be used to control the maximum torque applied to the screw to engage the coupling portion.

Intra-operative use means sensors and actuators which may be provided in or on the enhancer are preferably sterilizable or that part of the enhancer on which they are mounted can be packaged, similar to robotic applications. The material choice is subject to a similar requirement concerning sterilizability. Stainless steel (SS 316) or a titanium alloy (Ti6Al4V), both of which are used extensively in surgical instruments and have good sterilization properties, could be used.

The implant coupling portion 2 can be coupled to the implant by mounting into the conical Morse taper of the implant and can fixed using a screw, for example M7 screw.

The matching portion 3 of the enhancer 1 can allow to modify the vibrational response of the enhancer-implant-bone system. This can allow the modal strain energy distribution of the system to be altered. As was shown hereinbefore in the experimental and simulated results, the distribution of the modal strain energy has an important influence on the detectability of a contact change in a particular zone and changes in the critical proximal zone may only be detectable in the higher frequency range. It was also shown that the higher frequency range typically exhibits higher modal damping, thereby reducing its contribution to the FRF feature. In embodiments wherein one or more sensors or actuators are provided on or in the enhancer 1, dimensions of the implant coupling portion 2 can be chosen so as to accommodate these. However, in some embodiments sensors or actuators may not be provided in or on the enhancer 1 and may be provided separately to the enhancer and connected to the enhancer when required.

Common mounting thread sizes for accelerometers or actuation equipment range from 10-32 UNF to 1/4-28 UNF, corresponding in metric pitch sizes to an M5 x 0.8 and M6 x 1. By way of example, in some embodiments, to allow sufficient surface space to fix and tighten the sensors or actuators, an additional 1.5 mm around the threaded hole can be provided. This practical consideration translates to a minimum height and length of the implant coupling portion 2 of, for example, 9 mm.

The shape, size, and dimensions of the matching portion 3 can be chosen so as to change the modal strain energy distribution of the enhancer-implant-bone system so that the number of mode shapes which display high MSED values in the proximal region is increased (and thus will be sensitive to changes in this region) and optionally so that that this region is interrogated by modes in a lower frequency range to mitigate the influence of damping. Put differently, the implant-bone system may have a first frequency response function, and the enhancer-implant-bone system may have a second frequency response function, and the matching portion 3 may be configured such that the mode density of the second frequency response function in a frequency range is greater than the mode density of the first frequency response function in the same frequency range. The frequency range may have a lower frequency limit of 0 Hz, 100 Hz, 500 Hz, or intermediate or greater values. The frequency range may have an upper frequency limit of 500 Hz, 1 kHz, 2 kHz, 3 kHz, 4 kHz, 5 kHz, or intermediate or greater values. For example, the frequency range may be from 0 Hz to 2 kHz or from 0 Hz to 4.5 kHz. The skilled person will appreciate that other frequency limits and ranges are possible within the scope of the present invention and the present invention is not limited to the specific frequency limits and ranges disclosed herein.

The influence of geometrical changes to the implant-bone system can be seen in the following simulations. To reduce the complexity inherently present when working with biomechanical constructs, the bending and longitudinal behavior of a bone-implant system may be approximated by a simple beam model. For the results of this experiment, material properties nor geometry needed to correspond to those found for the bone-implant system. The model consists of 1000 linear beam elements, has a circular cross-section with a radius of 10 mm and a length of 1000 mm. Homogeneous material properties were chosen with an E-modulus of 100 GPa and a density of 4 g/cc. The first 13 flexible modes were calculated which resulted in two longitudinal mode shapes (L1,L2) and 11 bending mode shapes (B1-B11) spanning a range up to 5000 Hz.

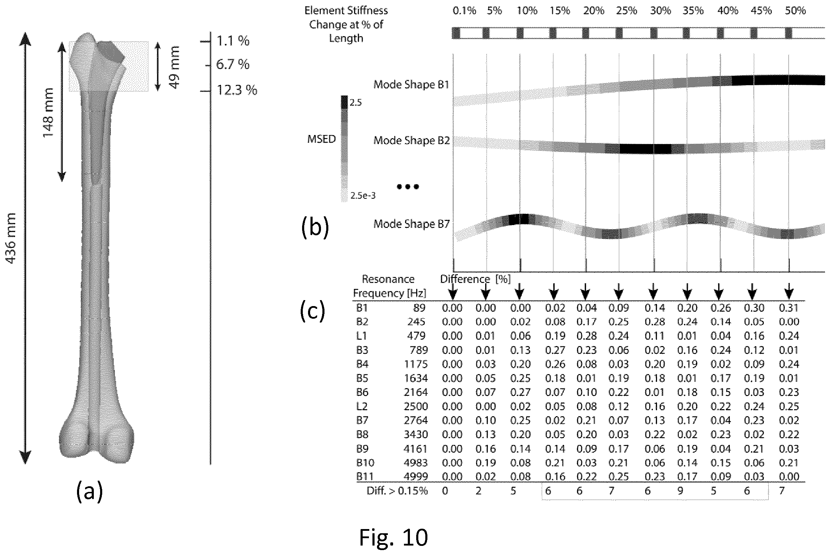

In addition, the MSED was calculated for every mode shape and is illustrated in Figure 10b. To establish a relation between changes to the system at certain locations and its effect on the resonance frequencies of the model, the stiffness of one element was reduced by 80% and the resonance frequencies of the altered model were calculated. Only one element was changed at a time, and its location was moved consecutively from the end of the beam model (at 0.1% of total length) towards the middle of the model (at 50% of total length) in 11 steps (at 0.1%, 5%, 10%, 15%, 20%, 25%, 30%, 35%, 40%, 45% and 50% of total length).

The effect of this change in stiffness on the resonance frequencies is provided in Figure 10c. It can be seen that resonance frequency changes are low despite a 80% local change in stiffness. In spite of this, the results again confirm that when a stiffness change is applied in a region close to a region with an elevated MSED for a particular mode, the change in resonance frequency of that mode is larger. The closer the change is applied to the region around 50 % of total length, the lower the frequencies that are affected. Also, the number of frequencies that are sensitive to a stiffness change increases as the location of the defect moves from the edge towards the middle of the beam system. Considering a threshold at 0.15% to flag a frequency as sensitive to the local change (which corresponds approx. to half of the maximum resonance frequency percentage change), an important increase of the number of sensitive resonance frequencies is observed when the change is present around the 10 - 15 % of total length region.

Referring to Figure 10a, these findings are translated to the femoral bone-implant system. Figure 10a illustrates the geometry of a Sawbones composite model in combination with a Wright size five implant. The crucial calcar zone in this example is at 6.7 % of total length and thus outside of the more sensitive region as was found for the beam model. Based on the simplified model, the region above the calcar zone is expected to be very little influenced by stiffness changes in the system (e.g. due to contact changes), whereas the region below is expected to be more sensitive. In this example, the total proximal zone comprises approx. 10 % of the total length of the bone and is measured from the first end 10 of the bone, that is, the end of the bone which is closest to the implant. However, the present invention is not limited thereto and a zone of interest other than the proximal zone may comprise a different proportion of the total length of the bone and may be located, for example, closer to the second end 11 of the bone. To increase the number of resonance frequencies sensitive to a change in contact, the calcar zone should be located at between 15 % and 50 % of total length. This implies that the full proximal zone is then located +- 5 % around this calcar zone. This observation can inform the choice of length of the implant coupling portion 2. Taking into account the length of 40 mm of the implant coupling portion, the additional length of the subsystem can vary between approx. five and 265 mm, totaling to 42 (calcar zone at 15 %) and 303 mm (calcar zone at 50 %). It is important to notice that this is an estimate of the length that can be added, as this discards any possible geometrical stiffening effects due to the shape of the enhancer as compared to a straight beam assumption as was used for this numerical experiment.

The properties of the matching portion 3 are preferably chosen such that changes to the vibrational behavior of the implant are observable on the enhancer. In some embodiments, the dimensions of the enhancer element may be chosen so as to provide dynamic coupling of the enhancer with the implant-bone system, which can allows efficient transfer of vibrational behavior of the bone-implant system to the enhancer. For example, the properties of the enhancer 1 may be chosen such that the first resonance frequency of the system formed by the implant and the implant coupling portion 2 is substantially the same as that of the matching portion 3, for example within 5%, within 10%, or within 20% of the first resonance frequency of the system formed by the implant and the implant coupling portion.

This means that the implant - enhancer vibrational behavior is preferably well coupled and the deformation of the system's mode shapes is preferably global, rather than local. Local, uncoupled behavior of the enhancer may lead to FRF feature results in which the spectrum is dominated by the local deformation patterns of the enhancer and is decoupled from changes in vibrational behavior of the implant it is intended to make observable. One way of achieving well coupled behavior is to match the impedance of the enhancer closely to the impedance of the system to which is it coupled. The dynamic impedance of mechanical structures is mainly characterized by the structure's resonance frequencies. Based on a free-free FE simulation of the Profemur size five and size six implant with only the implant coupling portion (assumed to be fabricated from stainless steel) attached, the first bending mode of this implant-coupling portion structure is found at an average resonance frequency of 1898 Hz in the AP direction and 1928 Hz in the ML direction.

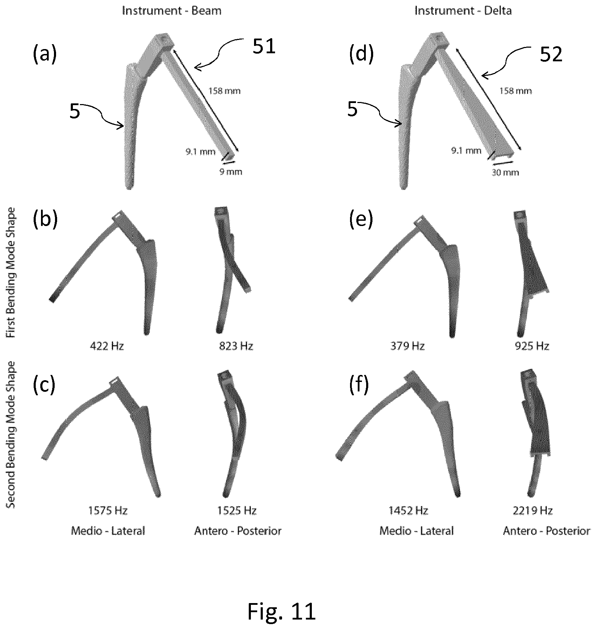

In some embodiments, referring to Figure 11a, the matching portion 3 can be shaped as a beam with rectangular cross section. A closed analytical formula is available providing the resonance frequencies of a beam given its dimensions and boundary conditions. This allows to precisely match these first resonance frequencies in both directions by modifying the length, width and height of this beam structure. To determine three parameters given two resonance frequencies conditions, one can be chosen freely. Setting the width to a value of 9 mm (which may in some embodiments be a limiting value as described hereinbefore), the length of the beam is solved to be 158 mm. The beam's resonance frequency of 1899 Hz thus matches the first AP bending mode of the implant-coupling portion structure at 1898 Hz. With a total enhancer length of 198 mm, the calcar zone would thus be located at 36 % from the top, which puts it comfortably in the sensitive target region as defined hereinbefore. Given this length, the height is then set at 9.14 mm, which results in a resonance frequency of 1928 Hz matching the ML bending mode of the implant-coupling portion structure at 1928 Hz. This approach leads to a shape of instrument which is very similar in size to the implant as it is in weight. The weight of the beam is 104 g and 58 g when manufactured from stainless steel or Ti6AL4V respectively.

A second test design adopted a delta shape for the matching portion 3 (Figure 11d). The length of the matching portion 3 as well as the height is kept the same for this design, but the width is changed. Rather than increasing the width of the beam over the whole length, a delta approach allowed to test for the influence of an increased stiffness in one direction without adding an excessive amount of weight. With a width of 30 mm at the wide end of the delta shape, its first bending frequency in the AP direction is predicted to be at 4091 Hz. Some additional modifications were made to the shape which lowered both the weight and the stiffness of the matching portion 3 (e.g. a recess was made in the center of the subsystem), resulting in a resonance frequency of 3616 Hz which is close to the second AP bending mode resonance frequency of the implant-coupling portion structure at 3616 Hz. Total weight of the delta matching portion is 171 g and 94 g when made from stainless steel or Ti6Al4V respectively.

The first bending mode shapes are illustrated in Figure 11b (beam) and 11e (delta). The second bending mode shapes are illustrated in Figure 11c (beam) and 11f (delta). Stainless steel material properties were assumed (E = 210 GPa, $\rho$ = 9 g/cc). The modal deformation shows that the design goal to develop an implant-instrument combination that is well coupled and shows global rather than local bending deformation patterns is well met by both designs. The similarity in impedance in the ML direction is substantiated by the closeness of their respective resonance frequencies, contrary to the bending behavior in the AP direction where the resonance frequencies, in particular for the second bending mode, are raised due to the increased design stiffness in this direction. The length of the matching portion of the enhancer was matched to couple to a size five and size six implant which resulted in the calcar zone located at a distance of 36 % from the end of the combined system. It is of interest to consider that for other bone lengths, this same length addition positions the calcar zone 38.9 % (bone length of 375 mm) and 33.9 % (bone length of 482 mm). For a wide range of bone sizes, this length addition thus ensures positioning the calcar zone in a sensitive MSED region.

Thus in embodiments of the present invention, the length of a matching portion of an enhancer element can be scaled to the length of the bone so as to position the location of a contact zone of interest at a distance which is a desired percentage of the total length of the bone-implant-enhancer system, as measured from the end of the bone-implant-enhancer system which is opposite to the enhancer. The percentage length may be for example within the range of 30% to 40% of the total length of the bone-implant-enhancer system, for example approximately 35% of the total length.

*In Silico Study*

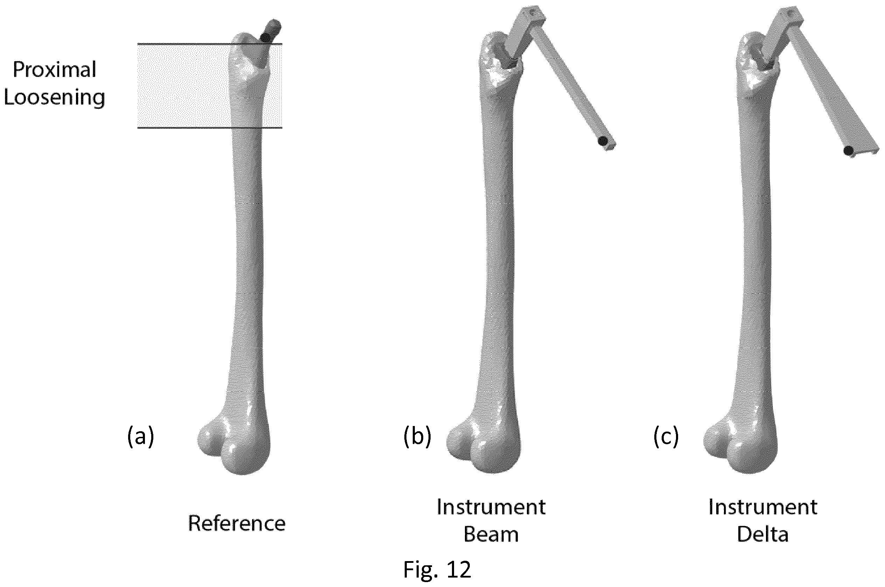

In order to assess the performance of the two instrument designs, FE models were built comprising bone, implant and enhancer. The vibrational behavior of these bone-implant-enhancer models was contrasted to the vibrational behavior of a reference bone-implant model. The models are depicted in Figure 12. Figure 12a shows the reference bone-implant model. Figure 12b shows the bone-implant-enhancer model for the beam form. Figure 12c shows the bone-implant-enhancer model for the delta form.

The enhancer-beam and enhancer-delta model consisted of 30209 and 76370 quadratic tetrahedral elements respectively. Two cases were simulated using these models. The first case considers the implant to be in full contact with the bone, the second case assumes a loss of contact in the proximal region, corresponding to zones 1-8.

The proximal contact area can have a large effect on primary stability of cementless implants and the enhancer according to embodiments of the present invention demonstrates high sensitivity to contact changes in this area. A modal analysis was performed on all models in the 10-10000 Hz range. A set of direct FRFs was synthesized at the virtual measurement points in the AP direction with a frequency resolution of one Hz. The FRFs of the two cases were compared using the Pearson's correlation metric. To understand the influence of including higher frequency information on the metric, PC values were calculated for ranges spanning 100-750 Hz to 100-10000 Hz with one Hz increments. To evaluate the instrument's design goal to increase the MSED in the proximal region in the lower frequency region, MSED distributions on the implant were calculated and contrasted to the MSED results for the reference model. Mechanical damping properties of the bone-implant construct can have an important effect on the shape of the FRF and thus on how frequency changes in the underlying system are reflected in this feature. To understand the influence modal damping has on the sensitivity of the FRF feature to contact changes, several modal damping scenarios were assumed for both cases. The scenarios with a modal damping of 0.5 %, 1.5 %; 5 % and 10 % considered damping to be the same for all modes in the 10-10000 Hz frequency range. Additionally, a scenario was added with a 2.5 % in the 10-2000 Hz range and 4.5 % in the range above 2000 Hz). This variation in modal damping with lower damping coefficients in the low frequency range and higher modal damping in the higher frequency range corroborates better with the experimental findings. Modal damping coefficients of 0.5 %-1.5 % are comparable to those found for composite bones, whereas damping coefficients of 4.5 %-5 % are comparable to those found in fresh frozen or cadaveric bones.

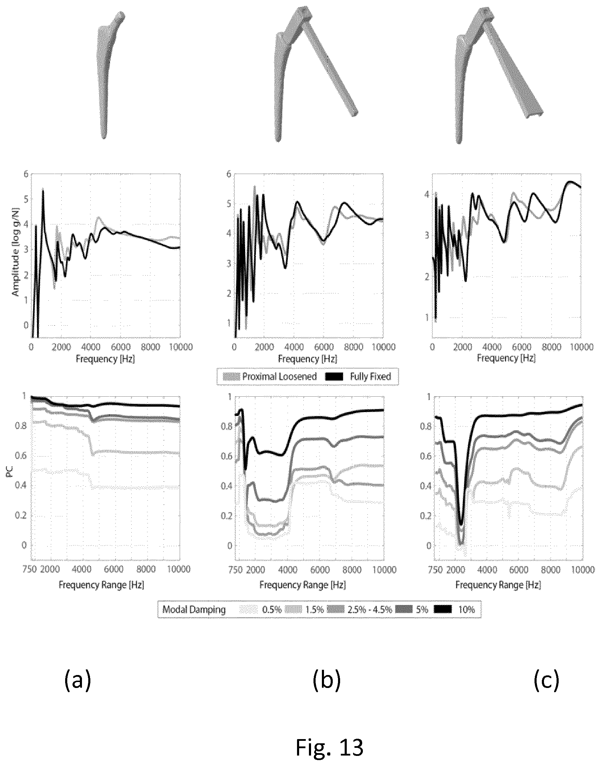

The results are presented in Figure 13 for the three models. Each column of Figure 13 shows, in order from the top of the figure, a schematic perspective view of the model used, the frequency response function for the proximally loosened and the fully fixed states, and the Pearson correlation at various damping levels. The metric values are calculated and plotted for varying ranges. E.g. the PC value plotted at 2000 Hz is the PC value calculated between the two fixation cases in the 100-2000 Hz range. Similarly, the value plotted at 4000 Hz is the PC value obtained for the 100-4000 Hz range etc. Rather than relying on a single PC value for a certain range, this representation gives insight into the sensitivity of the metric to the range selected. Lower values indicate higher sensitivity to contact changes in this area. In general, the metric values obtained for the implant-enhancer combinations are importantly lower than those of the reference model, except for the scenario with the lowest modal damping (0.5 %). Including the higher frequency range into the metric for the reference model improves the sensitivity of the metric, however this becomes less influential as damping is increased.

Although damping also affects the sensitivity of the implant-enhancer models, adequate performance of the metric is still expected to be present even in highly damped conditions and especially in the lower frequency region. The enhancer-delta design has better low frequency performance and lower minimal values than the enhancer-beam design when the range is extended to 2500 Hz, however the enhancer-beam design shows lower swings in sensitivity across the full frequency range. These results are summarized in Table 2.

*Table 2: Summary of the numerical experiment results for the reference, beam, and delta models. The average, maximal and minimal PC value is calculated on the 100-10000 Hz range. The average value is calculated for 100-1000 Hz range.*

| | Reference | | | | |
|---|---|---|---|---|---|
| Modal Damping [%] | 0,5 | 1,5 | 2,5 - 4,5 | 5,0 | 10,0 |
| *PC 10000 Hz Range* | | | | | |
| Mean (SD) | 0,43 (0,06) | 0,69 (0,09) | 0,86 (0,03) | 0,89 (0,04) | 0,95 (0,02) |
| Max | 0,95 | 0,97 | 0,99 | 1,00 | 1,00 |
| Min | 0,38 | 0,61 | 0,83 | 0,84 | 0,93 |
| *PC 1000 Hz Range* | | | | | |
| Mean (SD) | 0,58 (0,16) | 0,85 (0,05) | 0,93 (0,03) | 0,98 (0,01) | 0,99 (0,00) |
| | Instrument - Beam | | | | |
| Modal Damping [%] | 0,5 | 1,5 | 2,5 - 4,5 | 5,0 | 10,0 |
| *PC 10000 Hz Range* | | | | | |
| Mean (SD) | 0,27 (0,14) | 0,36 (0,18) | 0,40 (0,17) | 0,60 (0,18) | 0,80 (0,11) |
| Max | 0,47 | 0,71 | 0,79 | 0,86 | 0,91 |
| Min | 0,04 | 0,07 | 0,13 | 0,24 | 0,51 |
| *PC 1000 Hz Range* | | | | | |
| Mean (SD) | 0,28 (0,03) | 0,58 (0,02) | 0,70 (0,02) | 0,82 (0,01) | 0,88 (0,01) |
| | Instrument - Delta | | | | |
| Modal Damping [%] | 0,5 | 1,5 | 2,5 - 4,5 | 5,0 | 10,0 |
| *PC 10000 Hz Range* | | | | | |
| Mean (SD) | 0,23 (0,10) | 0,40 (0,14) | 0,59 (0,16) | 0,66 (0,17) | 0,81 (0,17) |
| Max | 0,38 | 0,66 | 0,83 | 0,86 | 0,94 |
| Min | -0,07 | -0,05 | 0,10 | 0,00 | 0,14 |
| *PC 1000 Hz Range* | | | | | |
| Mean (SD) | 0,13 (0,01) | 0,35 (0,01) | 0,49 (0,00) | 0,69 (0,00) | 0,86 (0,01) |

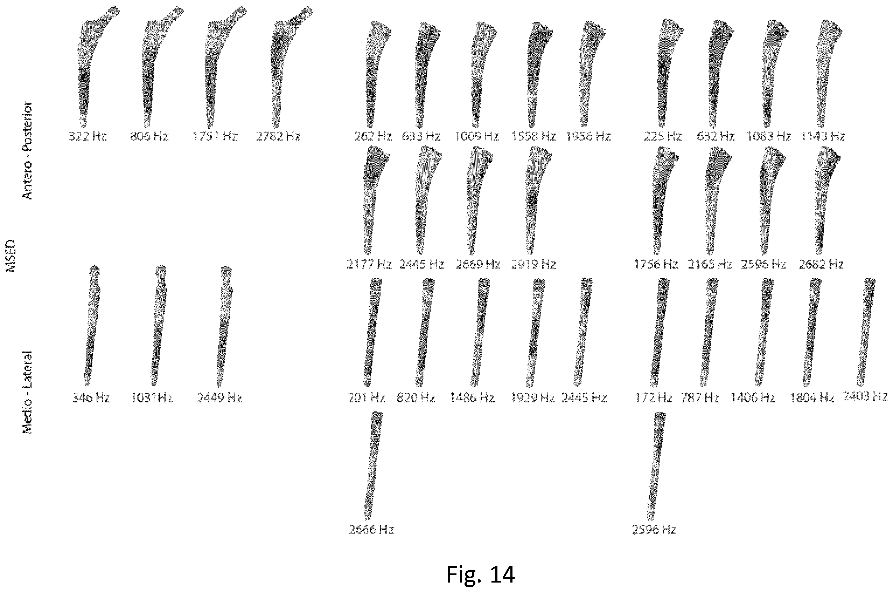

It can be seen that augmenting the implant with an enhancer indeed puts more frequencies in the lower frequency range and with resonance frequencies that are accompanied by mode shapes with important modal strain in the proximal region. A selection of the mode shapes in the 10-3000 Hz range with highest implant MSED are depicted for all three models in Figure 14. Darker shaded areas indicate regions with increased MSED distribution. Compared to the implant MSED distributions for the reference model, implant-enhancer MSED distributions in the proximal zone are found at lower frequencies (e.g. at 633 Hz for the enhancer -beam design and at 632 Hz for the enhancer-delta design) and for more mode shapes. The presence of more resonance frequencies (15 resonance frequencies in the 10-3000 Hz range for both enhancer models versus eight for the reference model) sensitive to changes in the proximal contact region observable in the FRF offers an explanation for the decrease of metric values observed.

Comparing the two enhancer designs, the following similarities and differences are noted. The length and bending stiffness in the ML direction of both designs are very similar. The total mass of the delta design is higher than the beam design as is the bending stiffness of the delta design in the AP direction.

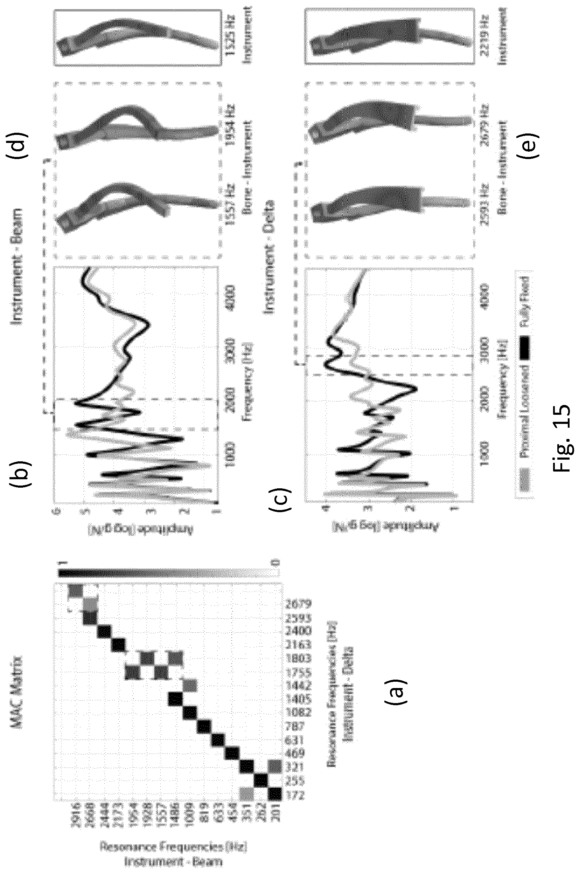

The FRFs for the two enhancer designs show many similarities, however some differences are observed. Firstly, the overall FRF amplitude of the enhancer -delta design is generally lower than that of the enhancer-beam design. Without wishing to be bound by theory, this could be due to the increased mass of the delta design, decreasing overall deformation amplitude for a certain input force. Secondly, two frequency ranges show different FRF behavior; the region around the 1557 Hz and 1954 Hz resonance frequencies of the enhancer -beam design and the region around the 2593 Hz and 2679 Hz resonance frequencies of the enhancer -delta design. These regions can be seen in Figures 15a and 15c marked by dashed lines. Figure 15b shows the FRF for the beam model and Figure 15c shows the FRF for the delta model. The highest FRF amplitude is also observed in these regions. Without wishing to be bound by theory, the sensitivity of the resonance frequencies to changes in contact in this region could be explained by the implant strain experienced as a result of the overall deformation of the bone-implant-enhancer model. This increase in local strain energy at the implant site may be a result of the altered strain distribution due to the added length of the augmented system. The high Modal Assurance Criterion values indicate that the mode shapes of the two bone-implant- enhancer systems show high resemblance, except in the two highlighted regions. Isolating the deformation the implant- enhancer experiences at these frequencies, it is noticed that the pattern shows large correspondence to the second bending AP mode of the free-free implant-enhancer system discussed hereinbefore. This is confirmed by a MAC value of 0.69 (at 1557 Hz) and to a lesser extent by the MAC value of 0.31 (at 1954 Hz) between the isolated bone-implant mode shapes found in the bone and their free-free second AP bending counterpart for the beam design. The MAC values calculated for the bone-implant-instrument systems are presented in a visual matrix format in Figure 15a. Figures 15d and 15e illustrate bending modes of the beam and delta configurations respectively, at the specified frequencies.

Likewise a MAC value of 0.60 (at 2593 Hz) and 0.66 (at 2679 Hz) is found for the implant-enhancer deformation in the bone and the second AP bending mode of the free-free implant-enhancer delta model. Furthermore the frequencies at which these patterns exhibit themselves in the bone-implant- enhancer model are in the vicinity or somewhat above the resonance frequency of the free-free implant- enhancer system. This holds for the region around 1500 Hz for the instrument-beam design and around 2500 Hz for the stiffer enhancer-delta design. As the implant- enhancer is thus deformed in a pattern and frequency close to the natural frequency of the free-free implant- enhancer, modal participation could be increased.

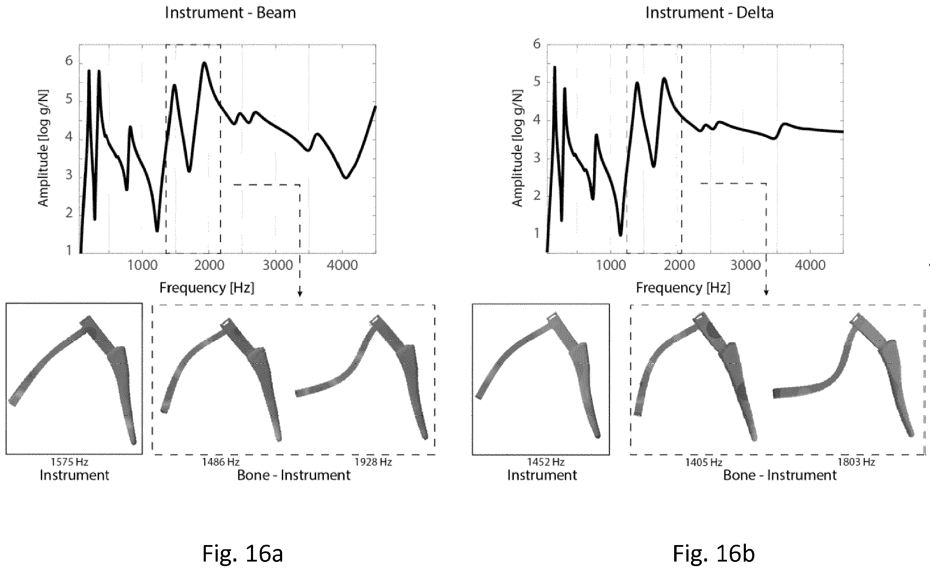

Referring to Figure 16, in the ML direction, a similar observation can be made. High modal participation of mode shapes to the FRFs (Figure 16a, beam; Figure 16b, delta) is observed in the region where the implant-instrument deformation in the bone resembles the free-free implant- enhancer deformation at comparable frequencies. Given similar impedances in the ML direction however, this increase in amplitude manifests itself in a similar frequency region.

*In Vitro Study*

An implantation process was performed in a composite femur model where after every insertion step three vibrational measurements of the system were taken; one on the bone-implant system without an enhancer, one with the instrument-beam design attached to the implant and one with the enhancer-delta design attached to the implant. This allowed to compare and contrast the evolution of the FRF feature for the different systems.

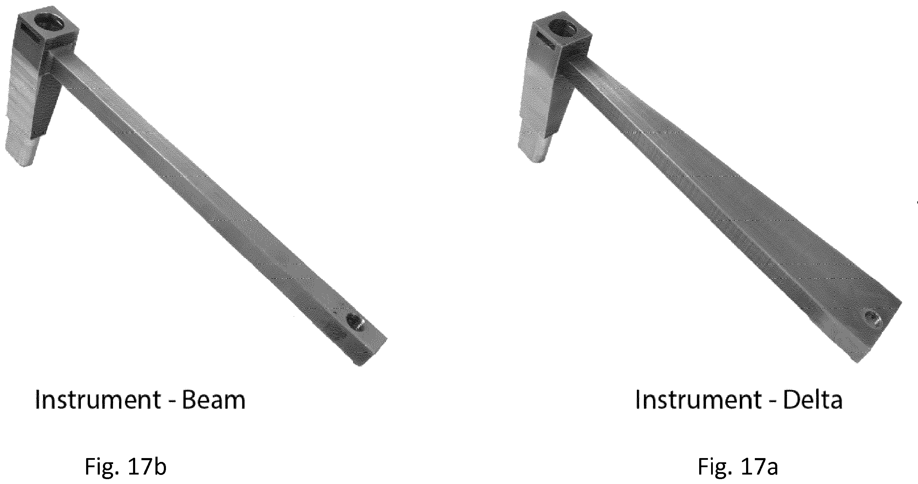

The two enhancer designs were manufactured using wire EDM (GF cut 300ms, AgieCharmilles, Geneva, Switzerland) and CNC milling (Kern Evo, Kern Microtechnik GmbH, Eschenlohe, Germany) from stainless steel alloy (SS 316). The resulting enhancers are depicted in Figure 17a (beam) and 17b (delta).

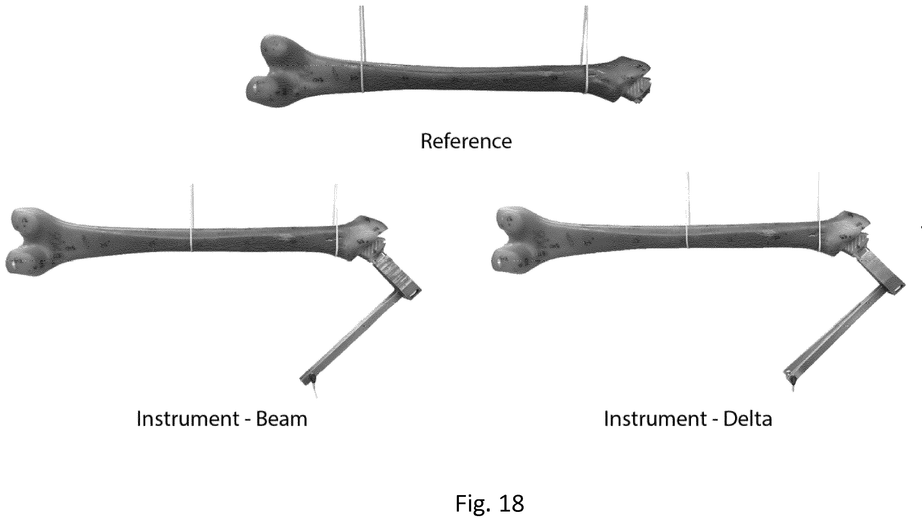

A composite femur model (Sawbones model 3403 (size medium), Sawbones Europe AB, Malmö, Sweden) was prepared by an experienced surgeon for implantation of an uncemented Profemur L size five implant using manufacturer provided standard instruments. After preparation, the implant was hammered in by the surgeon. After every insertion step, the depth of the implant was measured using a caliper. Three FRFs were collected after every insertion step; one on the bone-implant system (proximal edge of the Wright implant), one on the system with the enhancer-beam design mounted and one on the system with the enhancer-delta design mounted. The measurement points on the enhancers corresponded to the measurement points used in the in silico experiment. All FRFs were acquired in the AP direction. The excitation was performed by impact using a modal hammer (PCB 086C03) and the acceleration response was measured using a lightweight accelerometer (PCB A352A24). Data acquisition and conditioning was performed using a spectral analyzer (LMS SCADAS Mobile) and corresponding software (LMS Test Lab). The sampling frequency was set to 20.48 kHz, the frequency resolution was 0.625 Hz. Data processing was performed in Matlab. Free-free conditions were simulated. Figure 18 shows the bone-implant systems without and with the instruments mounted.

In addition to comparing the change in the FRFs using the PC an additional metric is introduced, the Frequency Response Assurance Criterion (FRAC). Corollary to the MAC used for mode shape comparison, the FRAC operates on the complex FRF vector rather than on the FRF magnitude as is the case for the PC.

$$FRAC = \frac{\left| \sum H_{pq}\left(f\right)_{N-1} H_{pq}{}^{*}\left(f\right)_{N} \right|^{2}}{\sum H_{pq}\left(f\right)_{N-1} H_{pq}{}^{*}\left(f\right)_{N-1} \sum H_{pq}\left(f\right)_{N} H_{pq}{}^{*}\left(f\right)_{N}}$$

Where $H_{pq}(f)_{N-1}$ is the FRF when the system is excited at location p and a response measurement is performed at location q for insertion step N-1. $H_{pq}(f)_N$ is the FRF measured and excited at the same locations for insertion step N.

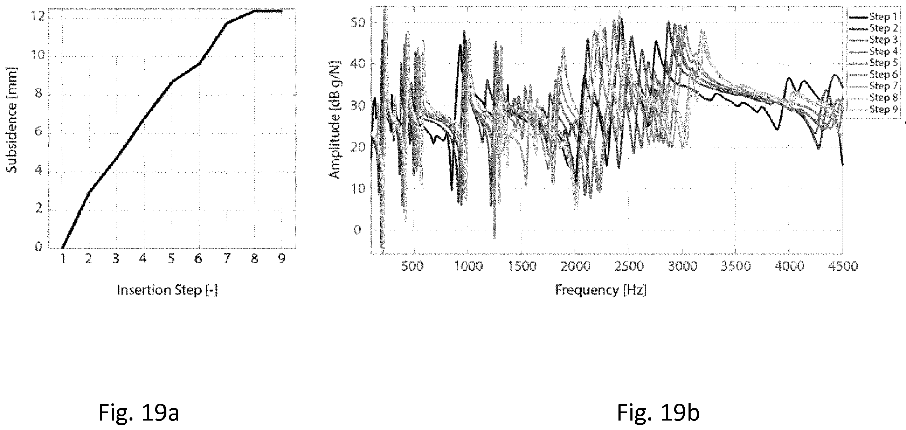

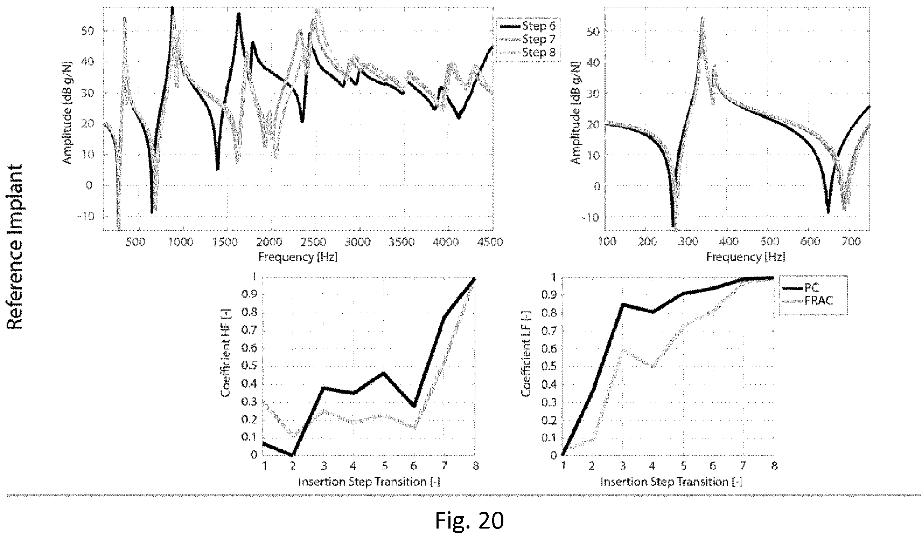

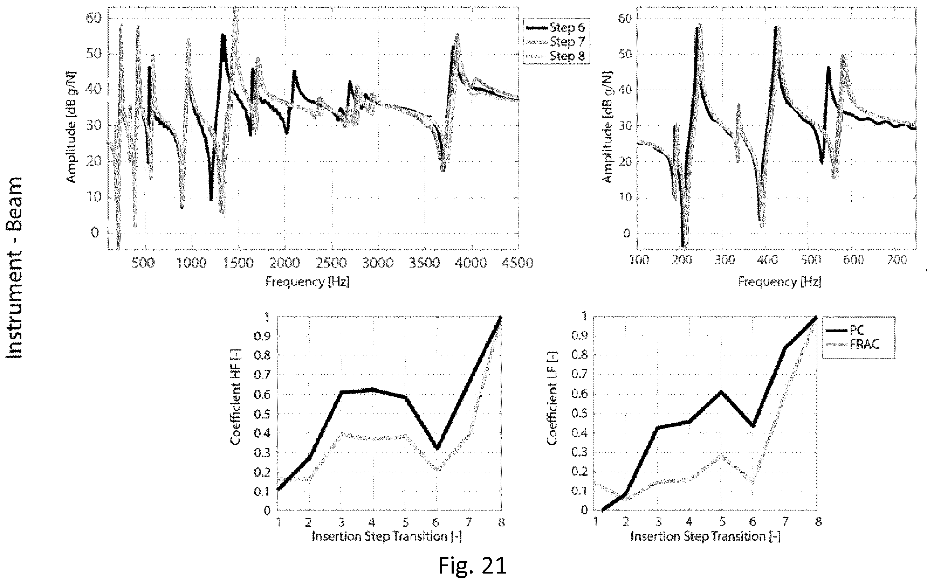

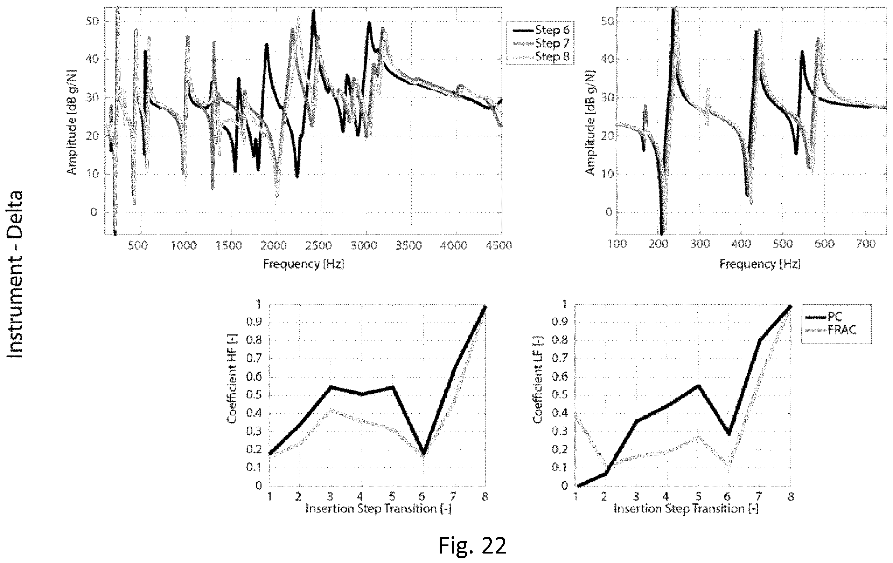

Figure 19a shows the evolution of the insertion depth or subsidence and, as an example, Figure 19b shows the FRFs for all steps of the enhancer-delta configuration. To compare the FRF evolution between the different configurations, Figures 20-22 illustrates the FRFs of steps six to eight of the insertion process for the reference (Figure 20), enhancer-beam (Figure 21) and enhancer-delta (Figure 22) experiments in a range of 100-4500 Hz and in zoomed on the LF behavior in the range 100-750 Hz. The PC and FRAC metrics obtained by comparing the FRFs of subsequent steps are presented for all three configurations, the first graph when the metric was calculated in a range 100-4500 Hz and zoomed in for the second graph with the metric calculated in the 10-750 Hz range, thus allowing to assess the influence and sensitivity of the high frequency versus the low frequency vibrational behavior to the insertion process. It can be seen from the frequency response functions that the mode density in the measured frequency range is increased for both systems which include the enhancer element according to embodiments of the present invention.

Metric values for all three configurations were generally low when the extended frequency range was considered (100-4500 Hz). The bone-implant reference configuration showed a high sensitivity to the changes in the insertion process. The metric values obtained by quantifying the change in the first six insertion steps (metric values one to five) are even below the metric values obtained for these same steps when compared to the enhancer-beam (an average of 0.08 lower for the FRAC metric and 0.19 lower for the PC metric ) or the enhancer-delta (an average of 0.08 lower for the FRAC metric and 0.19 lower for the PC metric). This changes however for the last three insertion steps (seven to nine). The difference reduces to 0.05 (FRAC metric) and 0.04 (PC metric) comparing step six to seven for the enhancer-beam and changes signs for steps seven to eight where the FRAC and PC metric are respectively lower by 0.14 and 0.11 than the reference configuration. A similar observation can be made when comparing the metric values for insertion steps seven to nine of the enhancer-delta configuration to the reference configuration. Quantifying the change from step six to seven, the FRAC metric is 0.01 above the reference configuration whereas the PC metric is 0.10 lower than the values obtained for the reference configuration. For step seven to eight, the FRAC and PC metric values are 0.07 and 0.12 below the values of the reference configuration.

The enhancer augmented systems thus show a higher sensitivity towards the end of the insertion process when proximal contact is established, and a lower sensitivity in the first stages of insertion. The reasons for this are likely twofold. Firstly, the system is highly undamped. It was shown in the numerical study that when little damping was simulated (0.5 %), the reference configuration showed a sensitivity comparable to that of the enhancer-beam and enhancer-delta configuration. Any increase in damping however was shown to have a disproportionate effect on this performance. Secondly, implant subsidence is important in the first few steps of the insertion process. This translates into a change in overall geometry of the bone-implant system, as the length of this combined system is changing (shortening). The percentage sensitivity of resonance frequencies associated with transversal bending modes of a simple beam model is approximately -2, which means that a one % change in length will cause a -2 % change in (all) resonance frequencies. Considering that the bone-implant-enhancer configuration is approximately 50 % longer than the bone-implant configuration, resonance frequency changes solely due to this geometrical change can be estimated to be roughly 50 % higher for the bone-implant configuration.

Whereas the implant-enhancer configurations only showed a higher sensitivity to the final changes in the insertion process compared to the reference configuration when an extended frequency range was considered for the undamped composite bone model, the performances were very different in the low frequency range (100-750 Hz). The first five instrument-beam FRAC and PC metrics are on average respectively lower by an amount of 0.23 and 0.27 compared to the reference configuration. These differences are even more important for the metric values at six and seven, with FRAC values lower by an amount of 0.67 and 0.37 and the PC values lower by an amount of 0.50 and 0.15. Similarly, the first five enhancer-delta metric values were lower by an average of 0.16 (FRAC) and 0.30 (PC) compared to the reference configuration. Metric values at steps six and seven were lower by 0.70 and 0.41 (FRAC) and by 0.65 and 0.20 (PC) compared to the reference configuration. The high metric values obtained for the reference configuration make discerning between the last few steps difficult. In contrast, the low values obtained for the enhancer-augmented configurations allow to easily discriminate between the penultimate and the ultimate step. The performance of the instrument-augmented configurations is very similar for the different frequency ranges used. The third mode shifting clearly in the low frequency region reflects the modified strain distribution the addition of the enhancer causes to the bone-implant system.

The choice of metric influences the information extracted by the method. Ideally, the differences reflected in the metric are as high as possible as long as the implant has not reached its final position and close to zero when the final position is reached. The FRAC metric values obtained were generally lower than the PC metric values in the steps leading up to the insertion endpoint by an average of 0.13 (SD 0.16) across all configurations and were comparable at insertion endpoint with an average difference of 0.004 (SD 0.001). Values for both were above 0.99 at the insertion endpoint.

Exploiting the richer information content available by processing the complex vectors as compared to only comparing one dimension of those same FRFs thus seems to be advantageous at most steps, without changing the insertion endpoint threshold value.

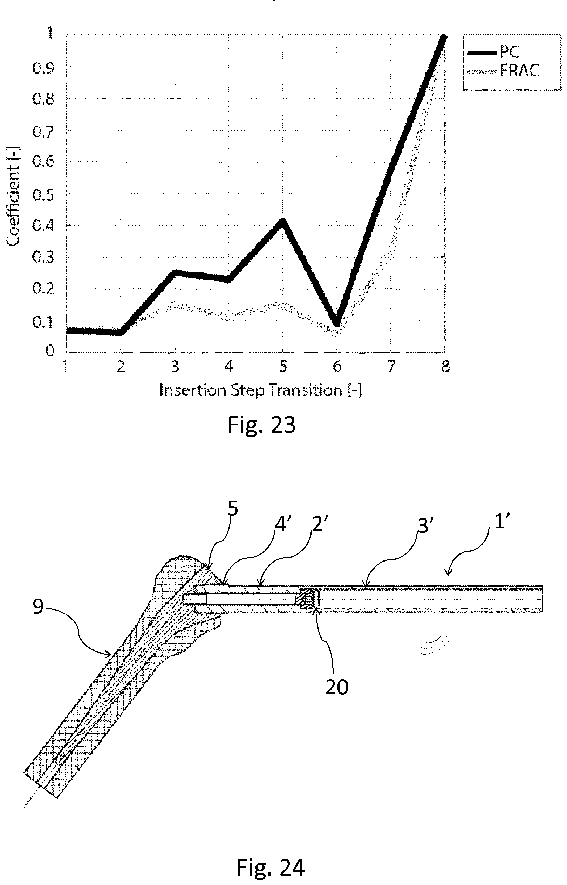

It is noted that the numerical study indicated a very sensitive region around 2500 Hz for the enhancer-delta design with an important shift of the resonance frequency when proximal contact was established. This same behavior is visible in the experimental measurements when the FRFs for the enhancer-delta design for steps six, seven and eight are investigated in the 2000 - 2500 Hz range. This validates the assumption that proximal contact is established in the final steps of the insertion process and confirms the relevance of the numerical cases simulated. In the extension of this, the sensitivity of the metric was shown to increase importantly when this region was included in the numerical study. When the enhancer-delta design metrics are indeed calculated for the range 100-2500 Hz, the metric values indeed are considerably lower, indicating an increased sensitivity to changes during the insertion process). This can be seen in Figure 23 which shows the PC and FRAC metrics as a function of insertion step transition when a range up to 2500 Hz was selected.

The increased sensitivity towards the end of the insertion allows to better differentiate between the penultimate and end step and thus for a better estimation of the insertion endpoint. The vibrational behavior in the low frequency region proved sensitive to proximal changes, which is especially of interest when damping in the system would increase. The observed behavior of the FRF and the similarity to the simulated cases furthermore confirms that proximal contact is indeed made in the last steps of the insertion process. Comparing the two enhancer designs, although both designs show adequate performance compared to the reference configuration, it was found that the enhancer-delta design was marginally more sensitive in the low frequency region.

Referring to Figure 24, a second enhancer element 1' according to embodiments of the present invention is shown. The second enhancer element 1' is an acoustic enhancer element. The second enhancer element 1' comprises a second implant coupling portion 2' and a second matching portion 3'. The second implant coupling portion 2' includes a second end portion 4' which is mechanically couplable to an orthopaedic implant 5. The second enhancer element 1' is configured to couple mechanically to an orthopaedic implant 5 at the second end portion 4' of the second enhancer element 1'. The mechanical coupling may be assisted by, for example, a screw or bolt attachment comprising a screw 20. The second enhancer element 1' is suitable for use in intraoperative assessment of implant stability in substantially the same manner as the first enhancer element 1, that is, by coupling to the implant 5 and being excited by an impact which does not substantially affect the stability of the implant within the bone. The second enhancer element 1' can form an enhancer-bone-implant system in substantially the same manner as the first enhancer element 1.

The second enhancer element 1' is designed to increase the acoustic radiation and increase the vibro-acoustic sensitivity of the (enhancer-)bone-implant system to bone-implant contact. By using the second enhancer element 1', which is couplable to the bone-implant system, the dynamic behavior of the bone-implant-enhancer system can be altered in order to increase the sensitivity to varying bone-implant contact area. The dimensions of the second enhancer element may be determined based on one or more criteria. For example, in some embodiments, the dimensions may be determined so as to increase the number of vibrational modes that are sensitive to a change in implant-bone contact area. By including the acoustic enhancer, the lower frequency modes (50-3000Hz) of the enhancer-implant-bone system show an increased modal strain energy density at the bone-implant interface which makes them more sensitive to changes in the contact area between implant and bone. Without the enhancer element, these similar modes for the bone-implant system are located at higher frequencies, which are more prone to damping and so more difficult to measure, and are less sensitive to changes at the bone-implant interface.

In some embodiments, the dimensions of the second enhancer element may be chosen so as to provide dynamic coupling of the enhancer with the implant-bone system, which can allow efficient transfer of vibrational behavior of the bone-implant system to the enhancer. For example, in some embodiments, the acoustic impedance of the enhancer element may be chosen as substantially similar to that of the implant. For example, the mass and the first resonance frequency of the enhancer may be chosen as substantially similar to those of the implant, for example within 1%, within 5%, or within 10% of the mass of the implant, and within 5%, within 10%, or within 20% of the resonance frequency of the implant. Hence, the mass of the enhancer may be within a range from 90% to 110% of the mass of the implant. For example, the mass of the enhancer may be the same as the mass of the implant. The enhancer element may be formed of a relatively less dense material, such as titanium rather than for example stainless steel, which can allow to provide a higher amplitude response and a correspondingly better acoustic radiation performance of the enhancer element. Thus, although the enhancer in embodiments of the present invention is not specifically adapted to support direct insertion hammer blows (which could actually damage the enhancer), its light structure is designed to augment the vibro-acoustic response of the enhancer-implant-bone system. The ratio Young's modulus/density, which is an important influencer of the vibrational behavior, is similar for both titanium and stainless steel, and so a lighter enhancer can be formed from titanium with similar vibrational behavior to stainless steel. Such a low damped metal alloy part can allow that the overall damping of the bone-implant-enhancer system is reduced. This can lead to an increased resolution of the measured frequencies of the enhancer-bone-implant system. Moreover, the enhancer is user-friendly, because a lighter instrument is easier to manipulate.

As described hereinbefore, a contact region may be defined by a region of contact between the implant outer surface and the bone cavity inner surface. In dependence on the type of implant and its properties, particular contact regions may be more sensitive to changes in implant fixation than other contact regions. An enhancer element according to embodiments of the present invention may be configured such that at least one vibrational mode of the enhancer-implant-bone system has an anti-node within a contact region of interest, so as to provide a relatively greater excitation in the contact region in comparison with an enhancer element not having a vibrational mode with an anti-node within the contact region.

The length of the enhancer element can be scaled to the length of the bone so as to position the location of a contact zone of interest at a distance which is a desired percentage of the total length of the bone-implant-enhancer system, as measured from the end of the bone-implant-enhancer system which is opposite to the enhancer. The percentage length may be for example within the range of 30% to 40% of the total length of the bone-implant-enhancer system, for example approximately 35%.

The dimensions of the second enhancer element are preferably chosen so as not to interfere with the working space of the orthopaedic surgeon and to provide an accessible location for application of the excitation impulse.

*Examples*

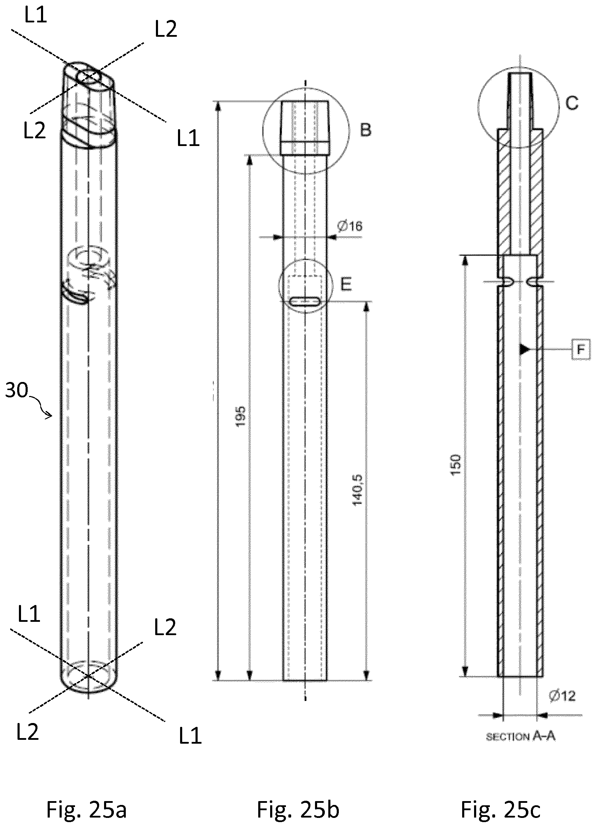

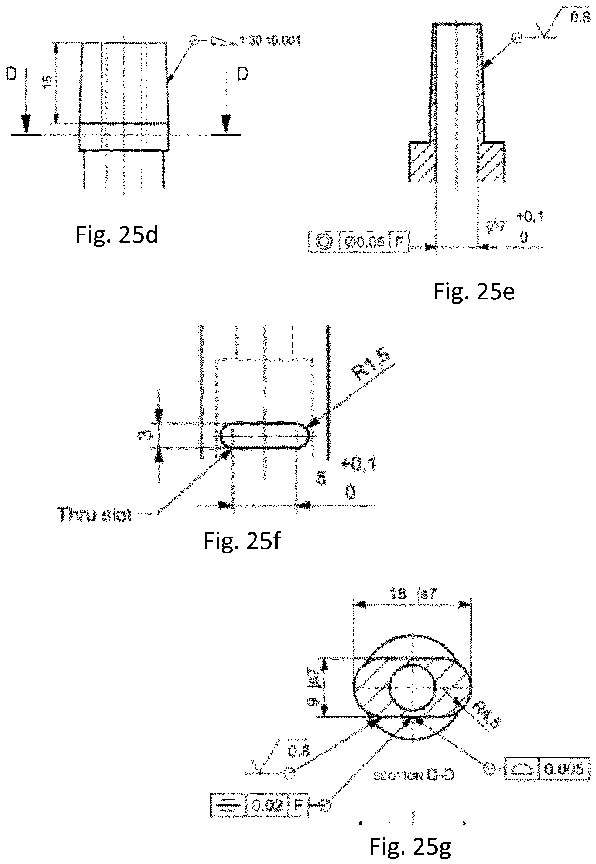

Referring to Figure 25a, a perspective view of an enhancer element 30 according to embodiments of the present invention is shown. Referring to Figure 25b, a cross-sectional view of the enhancer element 30 is shown in a plane indicated by L1-L1 in Figure 25a. Referring to Figure 25c, a cross-sectional view of the enhancer element 30 is shown in a plane indicated by L2-L2 in Figure 25a, being perpendicular to the plane of Figure 25b. Dimensions of the enhancer element are shown in mm, these being understood to be examples only and the present invention not being limited thereto. The detail of section B in Figure 25b is shown in Figure 25d. The detail of section C in Figure 25c is shown in Figure 25e. The detail of section The detail of section E in Figure 25b is shown in Figure 25f. A cross-section along the lines D-D of Figure 25d is shown in Figure 25g.

An in vitro study was done to assess the performance of the acoustic enhancer 30. A femoral stem (size 5, Gladiator, Microport) was inserted in a prepared artificial femur model (Sawbones, Malmo, Sweden). Two insertion experiments were performed with the use of the acoustic enhancer. Frequency response functions (FRF) and 'acoustic output only' measurements were done at every insertion step. During the FRF measurements the excitation force (input) is measured in order to normalize the response (output) using an instrumented hammer which included sensors for measuring the force of the hammer. During the 'acoustic output only' no reference measurement of the input force was done and used to normalize the measured output signals, only the acoustic response was measured during the hammer excitation, using a small hammer. The output only method has the advantage that there is no need for an instrumented hammer, which makes an in vivo implementation more feasible. The acoustic output in each case was measured using a microphone.

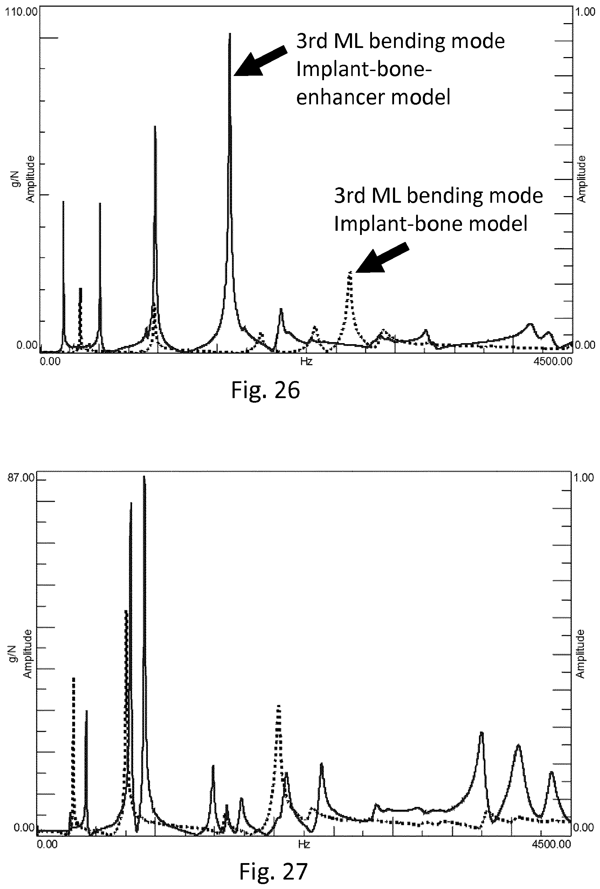

Experiment 1. To compare the vibrational behavior of the bone-implant system with and without the acoustic enhancer, FRFs were measured at the end of insertion with and without the acoustic enhancer. Figure 26 and 27 show the amplitude FRFs measured from the artificial bone-implant system (dashed line) and the bone-implant-enhancer system (solid line) in the range from 0 Hz to 4500 Hz. The implant was fully seated in the bone during the measurements, and measurements were taken in two perpendicular directions: medio-lateral (ML) Figure 26) and anterio-posterior (AP) (Figure 27). It can be seen from Figures 26 and 27 that the use of the enhancer increases the modal density in the frequency range from 0 to 4.5kHz: 20 modes with enhancer, 17 modes without enhancer. It can also be seen that there are more frequency modes in the lower frequency range (< 2000 Hz) and their amplitudes are higher, which can increase the measurability by providing a higher signal-to-noise ratio. The mode amplitudes are higher for most of the modes in the plotted frequency range with the enhancer as compared to without the enhancer, which is particularly visible in the lower frequency range (< 2000 Hz) in the ML direction and in the higher frequency range (3500Hz-4500Hz) in the AP direction. These higher amplitudes contribute to an increased acoustic measurability of the vibrational behavior of the bone-implant-enhancer system. This is especially the case in a system that is more damped than this example with artificial bone, as is the case in a real bone with soft tissue.

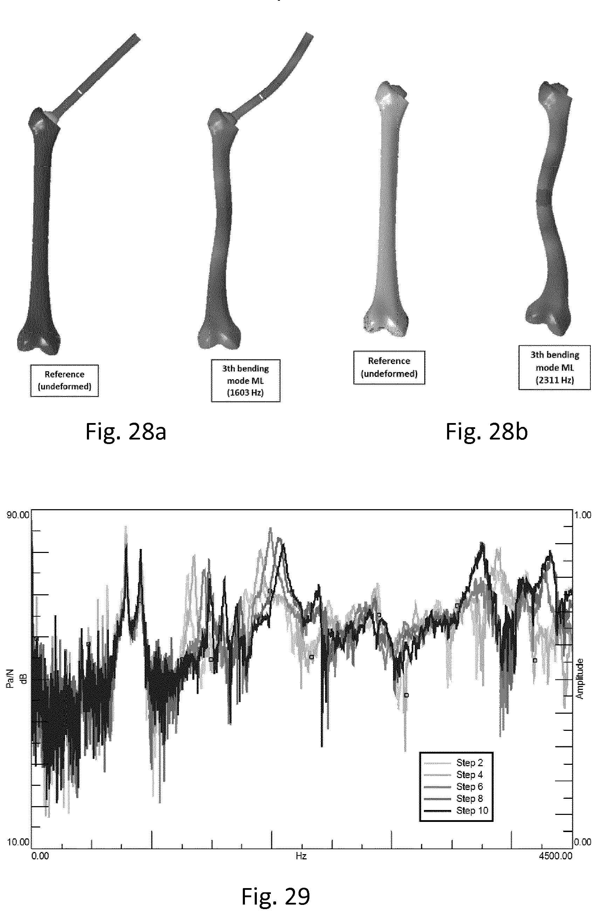

The shift of sensitive modes to a lower frequency can also be illustrated by a computer simulation. Figure 28 shows results of this in silico simulation. A bone model with (Figure 28a) and without enhancer (Figure 28b) are shown. For both cases, a system without deformation is shown on the left, and then the deformation at one mode, of the same system, is shown on the right. Figure 28 shows the deformation of the bone in an exaggerated way. It can be seen that at the location where the implant is located in the bone, there is an increased deformation, which makes this mode sensitive to a change in bone-implant contact. As can be seen in Figure 28, the frequency of this sensitive mode is lower when the enhancer is used, than without the use of this enhancer.

This frequency shift can also be noted in Figure 26, in which the 3rd ML mode is indicated for the bone-implant model and the corresponding 3rd ML mode for the bone-implant-enhancer model is shown. The 3rd ML mode for the bone-implant enhancer model is shifted to a lower frequency and has a higher amplitude than the corresponding mode for the bone-implant model. Other modes in the bone-implant-enhancer FRF are clearly shifted to lower frequencies than their corresponding modes in the bone-implant FRF.

Figure 29 illustrates the change in vibrational behavior of the bone-implant-enhancer system during the implant insertion experiment as measured in the AP direction. The acoustic FRFs measured at steps 2, 4, 6, 8, and 10 are shown . Multiple sensitive modes ($\pm$1500 Hz and $\pm$2000Hz in the AP direction) are well reflected in the acoustic response of the bone-implant-enhancer system. The shift of these frequencies during the insertion process can be seen clearly in this figure.

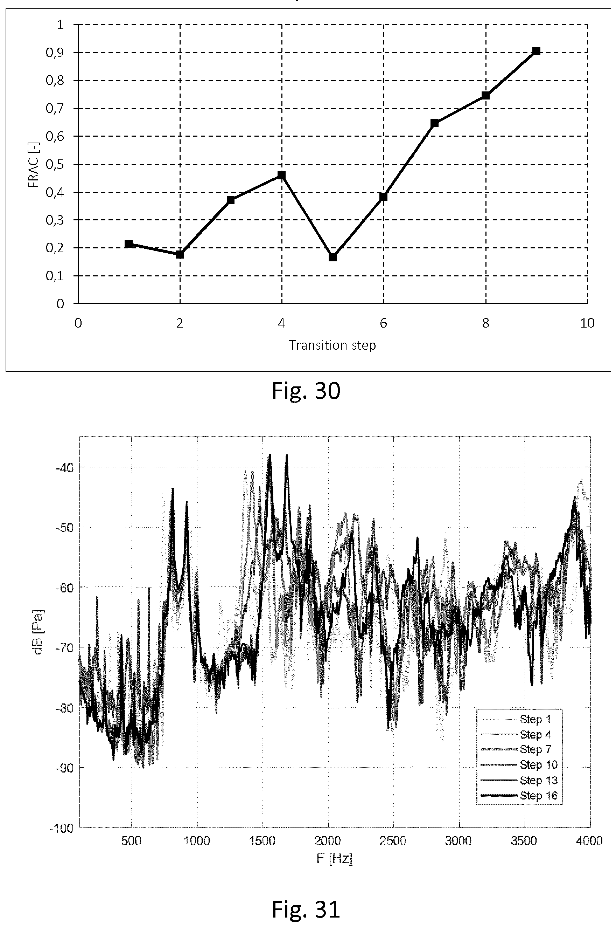

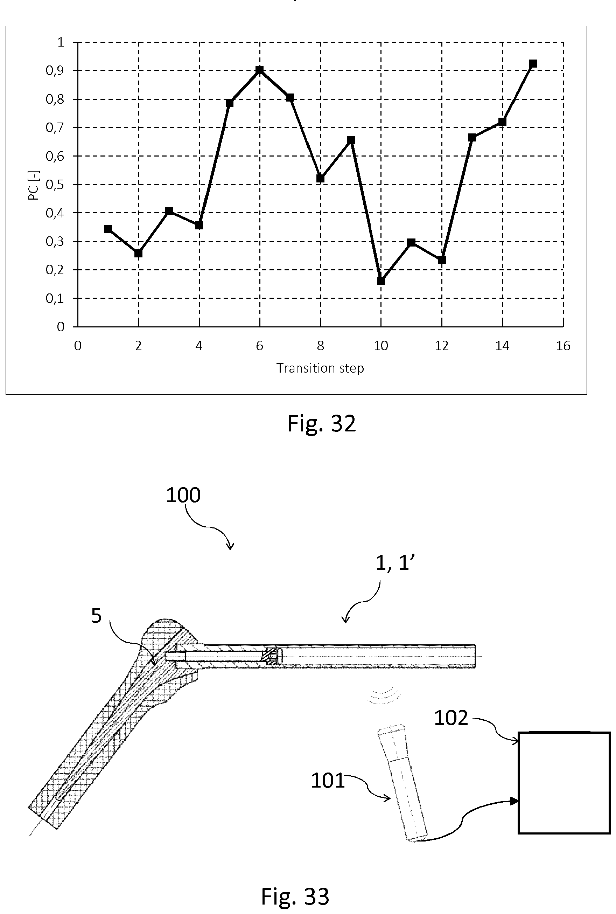

The Frequency Assurance Criterion (FRAC) was calculated as a modification metric. This index can be used to assess the change in the vibrational behavior of the system and detect the endpoint of insertion as described hereinbefore. Figure 30 shows that the FRAC metric evolves to a value above 0.9 when the implant was fully seated, indicating the endpoint of insertion. Experiment 2. Figure 31 shows acoustic frequency response functions measured during a second implant insertion experiment. During this experiment an acoustic output only measurement was used to calculate the frequency spectrum. Figure 32 shows the Pearson Correlation Coefficient metric (PC) calculated between every succeeding step of this implant insertion experiment.

Similar to the first experiment (using FRFs), the correlation metric evolves to a value above 0.9 indicating that the vibration behavior of the system stops changing once the endpoint of insertion is reached. During this experiment the instant of proximal contact is also visible in the evolution of the PC. Between transition step 10 and 12 the PC metric decreases, indicating an increased change of the vibrational behavior of the system caused by the increased proximal contact and press-fit during these steps.

The second enhancer element 1' can have one or more advantages. For example, an enhancer element can be provided which does not support any electronics or electrical elements, which can allow for easier sterilization and more cycles of sterilization during the lifetime of the element.

The acoustic measurements can have an integrative value: the acoustic signal contains the information from what is happening in the enhancer-implant-bone system as well as the surroundings, as opposed to the mechanical enhancer element wherein some information can be lost in the case that a measurement position of a detector is a position of zero or little mechanical displacement, such as a node.

Measurement of the acoustic signal can occur without needing to disturb the surgeon during the procedure, for example using a microphone (no measurement device is required to be attached/removed from the enhancer element).

Mechanical vibrations can be strongly dependent on soft tissue surroundings of the bone-implant system: these tend to weaken the signal, and the higher the frequency, the more it is prone to this damping effect. An enhancer element according to embodiments of the present invention can be used to shift the relevant frequencies to a better acoustically observable range (e.g. 1000-2500Hz).

Referring to Figure 33, a system 100 according to embodiments of the present invention is shown. The system 100 comprises an enhancer element 1, 1' according to embodiments of the present invention which is mechanically couplable to an implant 5, and a detector 101 configured to receive a vibrational signal from the enhancer element 1, 1'. The vibrational signal may be a mechanical vibration and/or an acoustic vibration. The detector may comprise, for example, a laser vibrometer, a microphone, an accelerometer, a velocity sensor. The detector 101 may be in physical contact or remote from the enhancer element 1, 1'. The detector 101 may be configured to provide signals to a processing module 102. The processing module 102 may be configured to receive signals from the detector 101 and to process such signals. For example, the processing module 102 may comprise a signal analyser. The processing module 102 may be configured to receive the raw input/output time data from the detector 101 and to calculate the frequency response function or output frequency spectrum out of which a modification index value as described hereinbefore can be calculated..

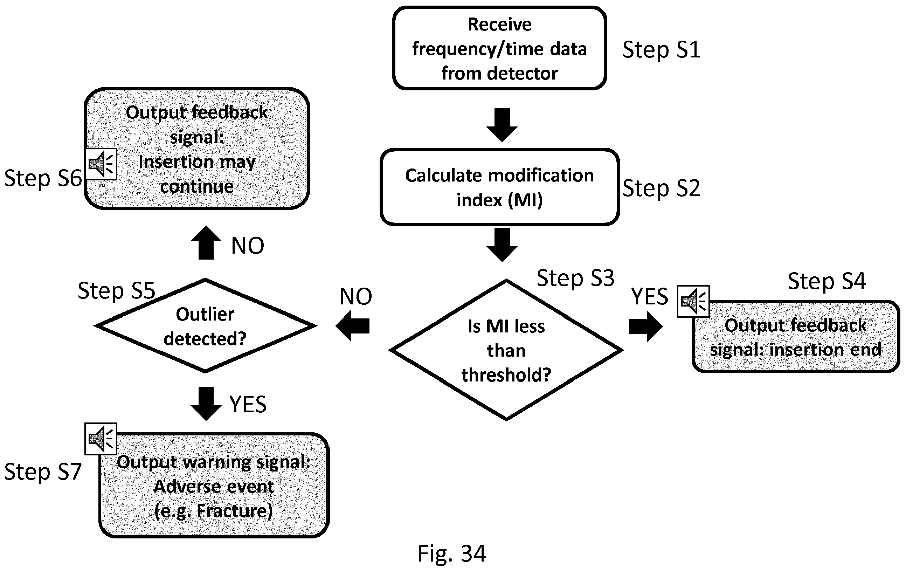

Referring to Figure 34, a flow chart of a method according to embodiments of the present invention is shown. The method is a method of determining an insertion end point, detecting a fracture risk, or determining a stopping point of insertion of an implant, by use of an enhancer element according to embodiments of the present invention. The method comprising receiving a frequency signal from a detector (step S1). A modification index is calculated based on the received frequency signal as described hereinbefore (step S2). The modification index (MI) is compared with a threshold value (step S3). The threshold value may be, for example, 0.1 or 0.01 but may be chosen to be any appropriate value, for example a value which indicates a sufficient degree of fixation of the implant. If the MI is less than the threshold value, a feedback signal, such as an audio signal or a visual indication on a screen, is provided to indicate that insertion may be halted (step S4).

If the MI is greater than the threshold value, an outlier detection step is carried out (step S5). If an outlier is not detected, a feedback signal, such as an audio signal or visual indication on a screen, is provided to indicate that insertion may continue (step S6). If an outlier is detected, for example as a discontinuity in the evolution of the MI as a function of insertion step, a feedback signal is provided to indicate that an adverse event such as a fracture has occurred or is imminent.

The method may be implemented using a computer program.

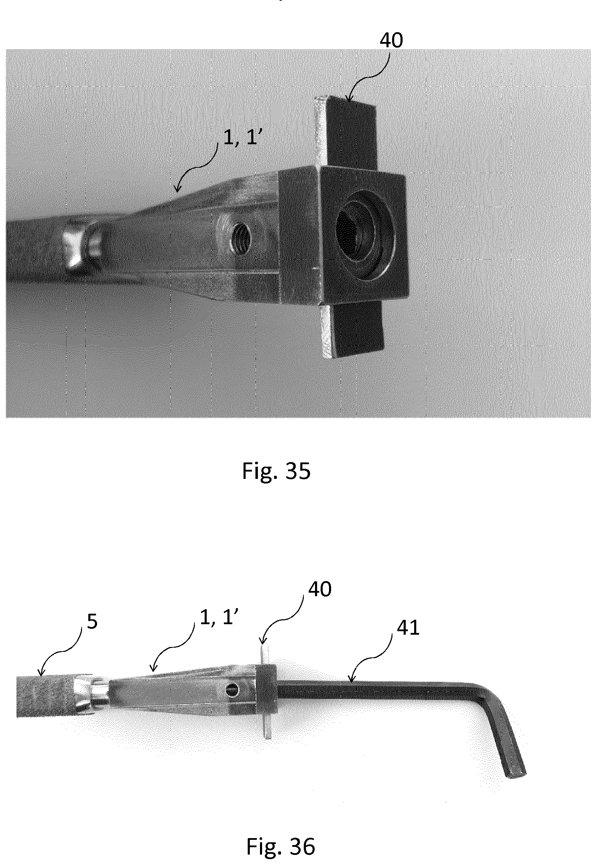

Referring to Figures 35 and 36, in some embodiments, the enhancer element 1, 1' can be coupled to the implant 5 using a connecting screw which is inserted into the implant. By unscrewing this screw, the screw moves away from the implant 5. A disengagement plate 40 may be provided in the enhancer, being a plate provided in a plane perpendicular to the axis of the screw, for example in a slot in the enhancer configured to prevent the plate from moving in the direction of the screw axis. Without the disengagement plate, the screw would move away from the implant along the screw axis, but the enhancer may remain in the implant as the end portion 4 of the implant coupling portion 2 may be tapered. This plate has a hole having dimensions so as to enable passing of the shaft of a screw driver 41, but the hole is too small to enable the head of the screw to pass, thus preventing further movement of the screw away from the implant. When the screw head makes contact with the plate, and is turned further, then the screw and the enhancer together will move away from the implant along the screw axis, hence the screw pushes out the enhancer from the implant.

The skilled person will appreciate that many modifications are possible within the scope of the present invention.

For example, the bone-implant system may be a cementless or a cemented bone-implant system.

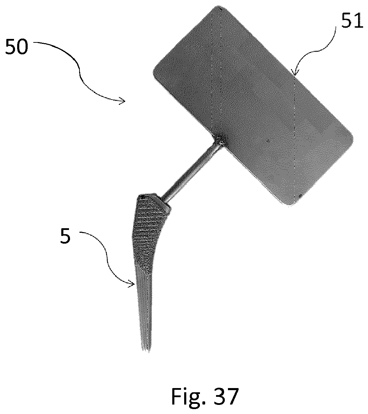

Referring to Figure 37, in some embodiments, the dimensions of a second enhancer element 50 may be chosen so as to provide a relatively large radiating surface 51, which can allow to increase the acoustic response during vibration.

The implant need not be a hip implant and may be for example an acetabular cup implant, a humeral implant, a glenoid implant, a tibial implant.

## Claims

1. A mechanical enhancer element (1) for use in intraoperative assessment of coupling of an orthopaedic implant (5) to a bone, wherein the implant and the bone form an implant-bone system having a first set of vibrational modes with a first mode density in a frequency range, wherein the enhancer element is adapted to be mechanically coupled to a first end of the orthopaedic implant (5) to form an enhancer-implant-bone system having a second set of vibrational modes with a second mode density in the frequency range, **characterized in that** the second mode density is greater than the first mode density, wherein the enhanced element is configured such that the first end of the orthopaedic implant is able to receive impaction blows for introducing the implant to the bone when the enhancer element is coupled thereto, wherein the vibrational response of the enhancer-implant-bone system during measurement of a vibrational mode, using a vibrational excitation that is separate from the impaction blows , is configured to provide information about the stiffness of the enhancer-implant-bone system, the enhancer element (1) being configured to enable assessment of the properties of an interface or contact region between the implant and the bone with increased sensitivity, compared to when the enhancer element (1) is not used.

2. An enhancer element (1) according to claim 1, wherein the first set of vibrational modes comprises a first vibrational mode, wherein the second set of vibrational modes comprises a second vibrational mode corresponding to the first vibrational mode, and wherein the second vibrational mode has a lower frequency than the first vibrational mode.

3. An enhancer element (1) according to any preceding claim, wherein the implant (5) has an implant mass, wherein the enhancer element (1) has an enhancer element mass similar to the implant mass within 10% of the implant mass, preferably equal to the implant mass.

4. An enhancer element (1) according to any preceding claim, wherein the implant (5) has an implant first resonance frequency, wherein enhancer element (1) has an enhancer element first resonance frequency which is substantially equal to the implant first resonance frequency.

5. An enhancer element (1) according to any preceding claim comprising an excitation element configured to provide a vibrational excitation to the enhancer-implant-bone system.

6. An enhancer element (1) according to any preceding claim comprising an excitation element configured to provide a vibro-acoustic excitation to the enhancer-implant-bone system.

7. An enhancer element (1) according to any preceding claim, wherein the frequency range includes frequencies from 10 Hz to 2,5 kHz or includes frequencies from 10 Hz to 5 kHz.

8. An enhancer element (1) according to any preceding claim, further comprising at least one sensor element disposed on the enhancer element (1) configured to detect a vibrational response of the enhancer-implant-bone system.

9.  An enhancer element (1) according to any preceding claim, wherein the implant (5) has an implant mechanical impedance, and wherein the enhancer (1) has an enhancer mechanical impedance which is substantially the same as the implant mechanical impedance.

10. An enhancer element (1) according to any preceding claim, wherein the implant (5) has an outer surface and the bone has a cavity for receiving the implant (5), the cavity having an inner surface, wherein a contact region is defined by a region of contact between the implant outer surface and the cavity inner surface, and wherein the second set of vibrational modes includes at least one vibrational mode having an anti-node within the contact region..

11. An enhancer element (1) according to any preceding claim, wherein the implant (5) is a cementless implant.

12. An enhancer element (1) according to any of claims 1 to 10, wherein the implant (5) is a cemented implant.

13. A system for intraoperative assessment of insertion of an orthopaedic implant comprising:
    a mechanical enhancer element (1) according to any preceding claim; and at least one detector configured to receive a vibrational signal from the enhancer element (1).

14. A system for intraoperative assessment of insertion of an orthopaedic implant comprising:
    a mechanical enhancer element (1) according to any of claims 1 to 12; and at least one detector configured to receive an acoustic signal from the enhancer element (1).

15. A system according to claim 13 or 14, wherein the at least one detector comprises at least one microphone.


**Patentansprüche**

1.  Ein mechanisches Verstärkerelement (1) zur Verwendung bei der intraoperativen Beurteilung der Kopplung eines orthopädischen Implantats (5) an einen Knochen, wobei das Implantat und der Knochen ein Implantat-Knochensystem bilden, das einen ersten Satz von Schwingungsmoden mit einer ersten Modendichte in einem Frequenzbereich aufweist, wobei das Verstärkerelement dazu ausgelegt ist, mechanisch mit einem ersten Ende des orthopädischen Implantats (5) gekoppelt zu werden, um ein Verstärker-Implantat-Knochensystem zu bilden, das einen zweiten Satz von Schwingungsmoden mit einer zweiten Modendichte in dem Frequenzbereich aufweist, **dadurch gekennzeichnet, dass** die zweite Modendichte größer ist als die erste Modendichte, wobei das verstärkte Element so konfiguriert ist, dass das erste Ende des orthopädischen Implantats in der Lage ist, Impaktionsschläge zum Einführen des Implantats in den Knochen aufzunehmen, wenn das Verstärkerelement daran gekoppelt ist, wobei die Schwingungsantwort des Verstärker-Implantat-Knochensystems während der Messung einer Schwingungsmode unter Verwendung einer Schwingungsanregung, die von den Impaktionsschlägen getrennt ist, so konfiguriert ist, dass sie Informationen über die Steifigkeit des Verstärker-Implantat-Knochensystems bereitstellt, wobei das Verstärkerelement (1) so konfiguriert ist, dass es eine Beurteilung der Eigenschaften einer Grenzfläche oder eines Kontaktbereichs zwischen dem Implantat und dem Knochen mit erhöhter Empfindlichkeit ermöglicht, verglichen damit, wenn das Verstärkerelement (1) nicht verwendet wird.

2.  Ein Verstärkerelement (1) nach Anspruch 1, wobei der erste Satz von Schwingungsmoden eine erste Schwingungsmode umfasst, wobei der zweite Satz von Schwingungsmoden eine zweite Schwingungsmode umfasst, die der ersten Schwingungsmode entspricht, und wobei die zweite Schwingungsmode eine niedrigere Frequenz als die erste Schwingungsmode aufweist.

3.  Ein Verstärkerelement (1) nach einem vorstehenden Anspruch, wobei das Implantat (5) eine Implantatmasse aufweist, wobei das Verstärkerelement (1) eine Verstärkerelementmasse aufweist, die ähnlich der Implantatmasse innerhalb von 10 % der Implantatmasse liegt, vorzugsweise gleich der Implantatmasse ist.

4.  Ein Verstärkerelement (1) nach einem vorstehenden Anspruch, wobei das Implantat (5) eine erste Implantatresonanzfrequenz aufweist, wobei das Verstärkerelement (1) eine erste Verstärkerelementresonanzfrequenz aufweist, die im Wesentlichen gleich der ersten Implantatresonanzfrequenz ist.

5.  Ein Verstärkerelement (1) nach einem vorstehenden Anspruch, umfassend ein Anregungselement, das so konfiguriert ist, dass es eine Schwingungsanregung für das Verstärker-Implantat-Knochensystem bereitstellt.

6. Ein Verstärkerelement (1) nach einem vorstehenden Anspruch, umfassend ein Anregungselement, das so konfiguriert ist, dass es eine vibroakustische Anregung für das Verstärker-Implantat-Knochensystem bereitstellt.

7. Ein Verstärkerelement (1) nach einem vorstehenden Anspruch, wobei der Frequenzbereich Frequenzen von 10 Hz bis 2,5 kHz beinhaltet oder Frequenzen von 10 Hz bis 5 kHz beinhaltet.

8. Ein Verstärkerelement (1) nach einem vorstehenden Anspruch, das weiter mindestens ein Sensorelement umfasst, das auf dem Verstärkerelement (1) angeordnet und so konfiguriert ist, dass es eine Schwingungsantwort des Verstärker-Implantat-Knochensystems detektiert.

9. Ein Verstärkerelement (1) nach einem vorstehenden Anspruch, wobei das Implantat (5) eine mechanische Implantatimpedanz aufweist, und wobei der Verstärker (1) eine mechanische Verstärkerimpedanz aufweist, die im Wesentlichen der mechanischen Implantatimpedanz entspricht.

10. Ein Verstärkerelement (1) nach einem vorstehenden Anspruch, wobei das Implantat (5) eine Außenfläche aufweist und der Knochen einen Hohlraum zur Aufnahme des Implantats (5) aufweist, wobei der Hohlraum eine Innenfläche aufweist, wobei ein Kontaktbereich durch einen Bereich des Kontakts zwischen der Außenfläche des Implantats und der Innenfläche des Hohlraums definiert ist, und wobei der zweite Satz von Schwingungsmoden mindestens eine Schwingungsmode mit einem Antiknoten innerhalb des Kontaktbereichs beinhaltet.

11. Ein Verstärkerelement (1) nach einem vorstehenden Anspruch, wobei das Implantat (5) ein zementfreies Implantat ist.

12. Ein Verstärkerelement (1) nach einem der Ansprüche 1 bis 10, wobei das Implantat (5) ein zementiertes Implantat ist.

13. Ein System zur intraoperativen Beurteilung des Einsetzens eines orthopädischen Implantats, umfassend:
ein mechanisches Verstärkerelement (1) nach einem vorstehenden Anspruch; und mindestens einen Detektor, der so konfiguriert ist, dass er ein Schwingungssignal von dem Verstärkerelement (1) empfängt.

14. Ein System zur intraoperativen Beurteilung des Einsetzens eines orthopädischen Implantats, umfassend:
ein mechanisches Verstärkerelement (1) nach einem der Ansprüche 1 bis 12; und mindestens einen Detektor, der so konfiguriert ist, dass er ein akustisches Signal von dem Verstärkerelement (1) empfängt.

15. Ein System nach Anspruch 13 oder 14, wobei der mindestens eine Detektor mindestens ein Mikrofon umfasst.

**Revendications**

1. Un élément amplificateur mécanique (1) pour utilisation dans l'évaluation intraopératoire du couplage d'un implant orthopédique (5) à un os, dans lequel l'implant et l'os forment un système implant-os ayant un premier ensemble de modes vibratoires avec une première densité de modes dans une plage de fréquences, dans lequel l'élément amplificateur est adapté pour être mécaniquement couplé à une première extrémité de l'implant orthopédique (5) pour former un système amplificateur-implant-os ayant un second ensemble de modes vibratoires avec une seconde densité de modes dans la plage de fréquences, **caractérisé en ce que** la seconde densité de modes est supérieure à la première densité de modes, dans lequel l'élément amplifié est configuré de telle sorte que la première extrémité de l'implant orthopédique est apte à recevoir des coups d'impact pour introduire l'implant dans l'os lorsque l'élément amplificateur y est couplé, dans lequel la réponse vibratoire du système amplificateur-implant-os lors de la mesure d'un mode vibratoire, en utilisant une excitation vibratoire qui est séparée des coups d'impact, est configurée pour fournir des informations sur la rigidité du système amplificateur-implant-os, l'élément amplificateur (1) étant configuré pour permettre l'évaluation des propriétés d'une interface ou région de contact entre l'implant et l'os avec une sensibilité accrue, comparée à lorsque l'élément amplificateur (1) n'est pas utilisé.

2. Un élément amplificateur (1) selon la revendication 1, dans lequel le premier ensemble de modes vibratoires comprend un premier mode vibratoire, dans lequel le second ensemble de modes vibratoires comprend un second mode vibratoire correspondant au premier mode vibratoire, et dans lequel le second mode vibratoire a une fréquence inférieure à celle du premier mode vibratoire.

3. Un élément amplificateur (1) selon toute revendication précédente, dans lequel l'implant (5) a une masse d'implant, dans lequel l'élément amplificateur (1) a une masse d'élément amplificateur similaire à la masse de l'implant dans 10% de la masse de l'implant, de préférence égale à la masse de l'implant.

4. Un élément amplificateur (1) selon toute revendication précédente, dans lequel l'implant (5) a une première fréquence de résonance d'implant, dans lequel l'élément amplificateur (1) a une première fréquence de résonance d'élément amplificateur qui est sensiblement égale à la première fréquence de résonance de l'implant.

5. Un élément amplificateur (1) selon toute revendication précédente comprenant un élément d'excitation configuré pour fournir une excitation vibratoire au système amplificateur-implant-os.

6. Un élément amplificateur (1) selon toute revendication précédente comprenant un élément d'excitation configuré pour fournir une excitation vibro-acoustique au système amplificateur-implant-os.

7. Un élément amplificateur (1) selon toute revendication précédente, dans lequel la plage de fréquences inclut des fréquences de 10 Hz à 2,5 kHz ou inclut des fréquences de 10 Hz à 5 kHz.

8. Un élément amplificateur (1) selon toute revendication précédente, comprenant en outre au moins un élément capteur disposé sur l'élément amplificateur (1) configuré pour détecter une réponse vibratoire du système amplificateur-implant-os.

9. Un élément amplificateur (1) selon toute revendication précédente, dans lequel l'implant (5) a une impédance mécanique d'implant, et dans lequel l'amplificateur (1) a une impédance mécanique d'amplificateur qui est sensiblement la même que l'impédance mécanique de l'implant.

10. Un élément amplificateur (1) selon toute revendication précédente, dans lequel l'implant (5) a une surface externe et l'os a une cavité pour recevoir l'implant (5), la cavité ayant une surface interne, dans lequel une région de contact est définie par une région de contact entre la surface externe de l'implant et la surface interne de la cavité, et dans lequel le second ensemble de modes vibratoires comprend au moins un mode vibratoire ayant un anti-nœud dans la région de contact.

11. Un élément amplificateur (1) selon toute revendication précédente, dans lequel l'implant (5) est un implant sans ciment.

12. Un élément amplificateur (1) selon l'une quelconque des revendications 1 à 10, dans lequel l'implant (5) est un implant cimenté.

13. Un système pour l'évaluation intraopératoire de l'insertion d'un implant orthopédique comprenant :

un élément amplificateur mécanique (1) selon toute revendication précédente ; et
au moins un détecteur configuré pour recevoir un signal vibratoire de l'élément amplificateur (1).

14. Un système pour l'évaluation intraopératoire de l'insertion d'un implant orthopédique comprenant :

un élément amplificateur mécanique (1) selon l'une quelconque des revendications 1 à 12 ; et
au moins un détecteur configuré pour recevoir un signal acoustique de l'élément amplificateur (1).

15. Un système selon la revendication 13 ou 14, dans lequel le ou les détecteurs comprennent au moins un microphone.

Fig. 1

Fig. 2

Fig. 3a

Fig. 3b

Fig. 4

Fig. 5

Fig. 6

Fig. 7

Fig. 8

Fig. 9

Fig. 10

Fig. 11

Proximal Loosening

(a) Reference

(b) Instrument Beam

(c) Instrument Delta

Fig. 12

(a)                              (b)                              (c)

Fig. 13

Fig. 14

Fig. 15

Fig. 16a

Fig. 16b

EP 3 817 690 B1

Instrument - Beam

Fig. 17b

Instrument - Delta

Fig. 17a

Reference

Instrument - Delta

Instrument - Beam

Fig. 18

Fig. 19b

Fig. 19a

Fig. 20

Fig. 21

Fig. 22

Fig. 23

Fig. 24

L1

L2

L2

L1

30

L1

L2

L2

L1

**Fig. 25a**

B

Ø16

E

195

140,5

**Fig. 25b**

C

F

150

Ø12

SECTION A-A

**Fig. 25c**

Fig. 25d

Fig. 25e

Fig. 25f

Fig. 25g

Fig. 26

Fig. 27

Fig. 28a

Fig. 28b

Fig. 29

Fig. 30

Fig. 31

Fig. 32

Fig. 33

```
                                         ┌─────────────────────┐
                                         │      Receive        │
                                         │ frequency/time data │  Step S1
                                         │   from detector     │
                                         └─────────────────────┘
                                                   │
                                                   ▼
  ┌─────────────────────┐                ┌─────────────────────┐
  │  Output feedback    │                │ Calculate modification │
  │      signal:        │                │     index (MI)      │  Step S2
  │  Insertion may      │                └─────────────────────┘
  │     continue        │                           │
  └─────────────────────┘                           ▼
Step S6                                     Step S3
           ▲ NO                                          YES    Step S4
   Step S5                    NO                                ┌──────────────────┐
        ◇ Outlier   ◀────────────   ◇  Is MI less  ─────▶      │ Output feedback  │
          detected?                    than                    │ signal: insertion end │
                                       threshold?              └──────────────────┘
                │ YES
                ▼
Step S7  ┌──────────────────────┐
         │ Output warning signal: │
         │    Adverse event      │
         │   (e.g. Fracture)     │
         └──────────────────────┘
```

Fig. 34

Fig. 35

Fig. 36

Fig. 37

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015187876 A1
- EP 3260088 A1
- US 20170196710 A1

**Non-patent literature cited in the description**

- **CRISTOFOLINI et al.** *Med. Eng. Phys.,* June 2006, vol. 28 (5), 475-82
- **QI et al.** How much can a vibrational diagnostic tool reveal in total hip arthroplasty loosening?. *Clinical Biomechanics,* vol. 18 (5), 444-458
- **LEURIDAN et al.** Determination of replicate composite bone material properties using modal analysis. *Journal of the Mechanical Behavior of Biomedical Materials,* vol. 66